# EUROPÄISCHE PATENTSCHRIFT

(11) **EP 3 596 066 B1**
(45) Veröffentlichungstag und Bekanntmachung des Hinweises auf die Patenterteilung: **18.05.2022**
(21) Anmeldenummer: 18710052.4
(22) Anmeldetag: 12.03.2018
(51) Int. Cl.: C07D 405/14, C07D 413/14, C07D 405/04, C07D 417/04, C07D 419/14, C07D 498/06, C07D 513/04, H01L 51/00, C07F 7/08

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 15.03.2017 EP 17160998
(43) Veröffentlichungstag der Anmeldung: 22.01.2020
(73) Patentinhaber: Merck Patent GmbH, 64293 Darmstadt (DE)
(72) Erfinder: PARHAM, Amir, 60486 Frankfurt Am Main (DE); EBERLE, Thomas, 76829 Landau (DE); JATSCH, Anja, 60489 Frankfurt Am Main (DE); GROSSMANN, Tobias, 64297 Darmstadt (DE); KROEBER, Jonas Valentin, 60311 Frankfurt Am Main (DE)
(86) Internationale Anmeldenummer: PCT/EP2018/055998
(87) Internationale Veröffentlichungsnummer: WO 2018/166934

(56) Entgegenhaltungen:
- WO-A1-2011/088877
- WO-A1-2016/015810
- WO-A2-2012/143080

## Beschreibung

Die vorliegende Erfindung beschreibt Dibenzofuran-und Dibenzothiophenderivate, insbesondere für die Verwendung als Triplettmatrixmaterialien in organischen Elektrolumineszenzvorrichtungen. Die Erfindung betrifft ferner ein Verfahren zur Herstellung der erfindungsgemäßen Verbindungen, sowie elektronische Vorrichtungen enthaltend diese Verbindungen.

In organischen Elektrolumineszenzvorrichtungen (OLEDs) werden als emittierende Materialien häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien, wie zum Beispiel Matrixmaterialien, von besonderer Bedeutung. Verbesserungen dieser Materialien können somit auch zu deutlichen Verbesserungen der OLED-Eigenschaften führen.

Gemäß dem Stand der Technik können eine Vielzahl unterschiedlicher Materialklassen als Matrixmaterialien für phosphoreszierende Emitter verwendet werden, unter anderem Carbazolderivate, Dibenzofuranderivate oder Triazinderivate.

Generell besteht bei Materialien für die Verwendung als Matrixmaterialien noch Verbesserungsbedarf, insbesondere in Bezug auf die Lebensdauer, aber auch in Bezug auf die Effizienz und die Betriebsspannung der Vorrichtung.

WO 2012/143080 und WO 2011/088877 offenbaren verbrückte aromatische Amine für die Verwendung in organischen Elektrolumineszenzvorrichtungen. Aus WO 2016/015810 sind Dibenzofuran- und Dibenzothiophenderivate für die Verwendung in organischen Elektrolumineszenzvorrichtungen bekannt, welche in 1-Position mit einer Heteroarylgruppe und in 8-Position mit einer Diarylamino- oder N-Carbazolylgruppe substituiert sind.

Aufgabe der vorliegenden Erfindung ist die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen.

Es wurde überraschend gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung gemäß der folgenden Formel (1), 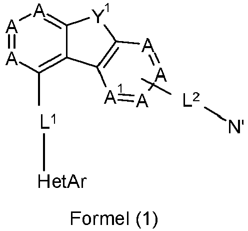 wobei für die verwendeten Symbole gilt:
- A, A¹: ist bei jedem Auftreten gleich oder verschieden CR¹ oder N, wobei von allen A und A¹ maximal zwei Gruppen A und/oder A¹ pro Cyclus für N stehen und jenes A für C steht, an welches L² gebunden ist;
- Y¹: ist O, S oder NR, wobei der Rest R, der an N gebunden ist, ungleich H oder D ist;
- L¹: ist eine Einfachbindung oder ein aromatisches oder heteroaromatisches Ringsystem mit 5 bis 30 aromatischen Ringatomen, das durch einen oder mehrere Reste R³ substituiert sein kann;
- L²: ist eine Einfachbindung oder ein aromatisches oder heteroaromatisches Ringsystem mit 5 bis 30 aromatischen Ringatomen, das durch einen oder mehrere Reste R³ substituiert sein kann;
- N': ist eine Gruppe der Formel (L-1), 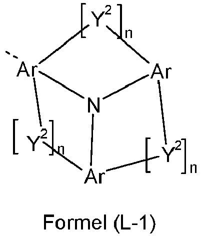 wobei die gestrichelte Bindung die Verknüpfung mit L² darstellt;
- Y²: ist bei jedem Auftreten gleich oder verschieden gewählt aus einer Einfachbindung, C(R)₂, C=O, O, S, S=O, SO₂, Si(R)₂, NR, wobei der Rest R, der an N gebunden ist, ungleich H oder D ist;
- n: ist bei jedem Auftreten gleich oder verschieden 0 oder 1, wobei wenn mindestens ein Y² für eine Einfachbindung steht, mindestens zwei n gleich 1 sind; dabei bedeutet n = 0, dass die entsprechende Gruppe Y² nicht vorhanden ist;
- Ar: ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann;
- HetAr: ist eine Gruppe der Formel (2), (3) oder (4), 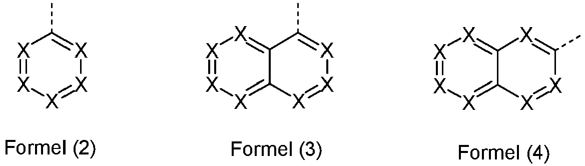 wobei die gestrichelte Bindung die Verknüpfung dieser Gruppe darstellt;
- X: ist bei jedem Auftreten gleich oder verschieden CR² oder N mit der Maßgabe, dass mindestens ein Symbol X für N steht;
- R, R¹, R², R³: ist bei jedem Auftreten gleich oder verschieden ausgewählt aus der Gruppe bestehend aus H, D, F, Cl, Br, I, CN, NO₂, N(Ar¹)₂, N(R⁴)2, C(=O)Ar¹, C(=O)R⁴, P(=O)(Ar¹)₂, P(Ar¹)₂, B(Ar¹)₂, Si(Ar¹)₃, Si(R⁴)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, Si(R⁴)₂, C=O, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei benachbarte Substituenten R, bzw. zwei benachbarte Substituenten R¹, bzw. zwei benachbarte Substituenten R², bzw. zwei benachbarte Substituenten R³ ein aliphatisches, heteroaliphatisches, aromatisches oder heteroaromatisches Ringsystem bilden, das mit einem oder mehreren Resten R⁴ substituiert sein kann;
- Ar¹: ist bei jedem Auftreten gleich oder verschieden ein aromatisches oder heteroaromatisches Ringsystem mit 5 bis 30 aromatischen Ring-atomen, das mit einem oder mehreren nicht-aromatischen Resten R⁴ substituiert sein kann; dabei können zwei Reste Ar¹, welche an dasselbe N-Atom, P-Atom oder B-Atom binden, auch durch eine Einfachbindung oder eine Brücke, ausgewählt aus N(R⁴), C(R⁴)₂, O oder S, miteinander verbrückt sein;
- R⁴: ist bei jedem Auftreten gleich oder verschieden ausgewählt aus der Gruppe bestehend aus H, D, F, Cl, Br, I, CN, NO₂, N(R⁵)₂, C(=O)R⁵, Si(R⁵)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, Si(R⁵)₂, C=O, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei benachbarte Substituenten R⁴ ein aliphatisches oder aromatisches oder heteroaromatisches Ringsystem bilden, das mit einem oder mehreren Resten R⁵ substituiert sein kann;
- R⁵: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch eine oder mehrere Alkylgruppen mit jeweils 1 bis 4 Kohlenstoffatomen substituiert sein kann; dabei können zwei oder mehr benachbarte Substituenten R⁵ miteinander ein aliphatisches Ringsystem bilden;
wobei folgende Verbindungen von der Erfindung ausgenommen sind:

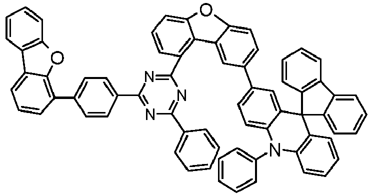

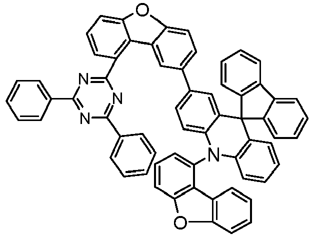

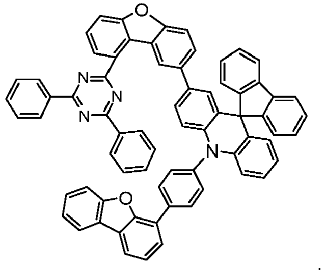

Bei den Verbindungen nach Formel (1) ist L² immer mit einer Gruppe A verbunden, nicht mit einer Gruppe A¹.

Benachbarte Substituenten im Sinne der vorliegenden Erfindung sind Substituenten, die an Kohlenstoffatome gebunden sind, die direkt miteinander verknüpft sind, oder die an dasselbe Kohlenstoffatom gebunden sind.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Dies wird durch das folgende Schema verdeutlicht:

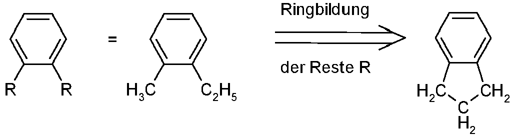

Weiterhin soll unter der oben genannten Formulierung auch verstanden werden, dass für den Fall, dass einer der beiden Reste Wasserstoff darstellt, der zweite Rest unter Bildung eines Rings an die Position, an die das Wasserstoffatom gebunden war, bindet.

Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Ein aromatisches Ringsystem im Sinne dieser Erfindung enthält 6 bis 40 C-Atome im Ringsystem. Unter einem aromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom, verbunden sein können. So sollen beispielsweise auch Systeme wie Fluoren, 9,9'-Spirobifluoren, 9,9-Diarylfluoren,Triarylamin, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Weiterhin werden miteinander durch Einfachbindung verknüpfte Aromaten, also Oligoarylene bzw. Oligoheteroarylene, wie zum Beispiel Biphenyl, Terphenyl oder Quaterphenyl, als aromatische Ringsysteme im Sinne dieser Anmeldung bezeichnet.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy oder 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 40 aromatischen Ringatomen werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Chinoxalin, Chinazolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von einer Kombination dieser Systeme.

In einer bevorzugten Ausführungsform der Erfindung steht maximal eine Gruppe A oder A¹ pro Cyclus für N, und die anderen Gruppen A und A¹ stehen für CR¹. Besonders bevorzugt stehen A und A¹ für CR¹, so dass es sich bei der Verbindung der Formel (1) um eine Verbindung der folgenden Formel (1a) handelt, 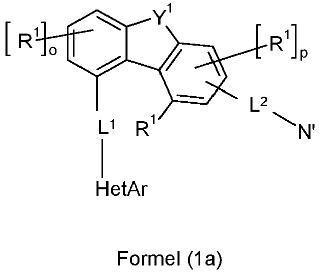 wobei die verwendeten Symbole die oben genannten Bedeutungen aufweisen, o für 0, 1, 2 oder 3 steht und p für 0, 1, oder 2 steht.

In einer bevorzugten Ausführungsform der Erfindung stehen die Indices p und o in Formel (1a) gleich oder verschieden für 0, 1 oder 2, besonders bevorzugt 0 oder 1 und ganz besonders bevorzugt für 0.

Bevorzugte Ausführungsformen der Formel (L-1) sind die Strukturen der Formeln (L-2) bis (L-7), 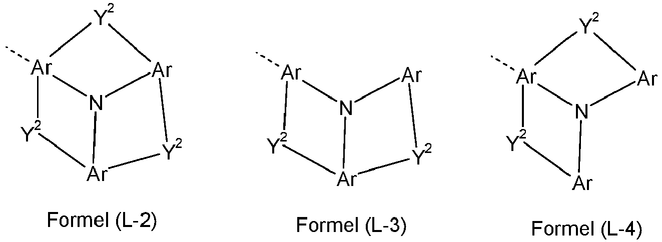 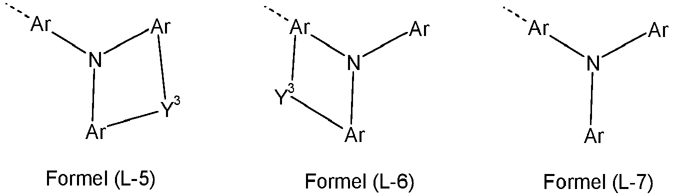 wobei die auftretenden Symbole die oben genannten Bedeutungen aufweisen und zusätzlich gilt:
- Y³: ist bei jedem Auftreten gleich oder verschieden gewählt aus C(R)₂, C=O, O, S, S=O, SO₂, Si(R)₂ oder NR, wobei der Rest R, der an N gebunden ist, ungleich H oder D ist.

Dabei stehen in Formel (L-2) bevorzugt nicht alle drei Gruppen Y² für eine Einfachbindung.

In einer weiteren Aufführungsform der Erfindung steht Y³ in Formel (L-5) nicht für C(Ph)₂, wobei die beiden Phenylgruppen über eine Einfachbindung miteinander verbunden sind.

In einer weiteren Ausführungsform der Erfindung ist Y³ bei jedem Auftreten gleich oder verschieden gewählt aus C=O, O, S, S=O, SO₂, Si(R)₂, NR, wobei der Rest R, der an N gebunden ist, ungleich H oder D ist.

In einer weiteren Ausführungsform der Erfindung ist Y³ gewählt aus C=O, O, S, S=O, SO₂, Si(R)₂ oder NR, wobei der Rest R, der an N gebunden ist, ungleich H oder D ist, und Y² ist bei jedem Auftreten gleich oder verschieden gewählt aus einer Einfachbindung, C=O, O, S, S=O, SO₂, Si(R)₂ oder NR, wobei der Rest R, der an N gebunden ist, ungleich H oder D ist.

In einer weiteren Ausführungsform der Erfindung ist Y³ gewählt aus O oder S, und Y² ist bei jedem Auftreten gleich oder verschieden ausgewählt aus einer Einfachbindung, O, S oder SiR₂.

In einer bevorzugten Ausführungsform der Erfindung steht N' für eine Gruppe der Formel (L-7).

Bevorzugte Ausführungsformen der Formel (1a) sind die Verbindungen der Formeln (1b) bis (1d), 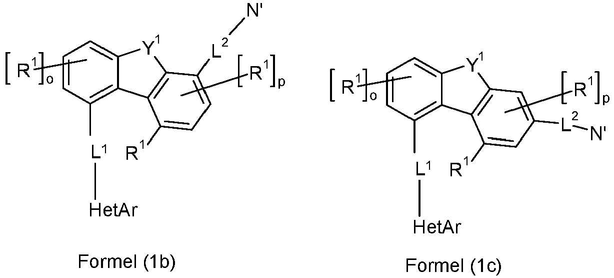 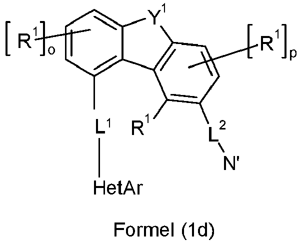 wobei die auftretenden Symbole und Indizes die vorstehenden Bedeutungen aufweisen.

Eine besonders bevorzugte Ausführungsform ist die Verbindung der Formel (1d).

Im Folgenden werden bevorzugte Ausführungsformen der Gruppe HetAr beschrieben.

Bevorzugte Ausführungsformen der Gruppen der Formeln (2), (3) und (4) sind die Gruppen der folgenden Formeln (2-1) bis (2-10), (3-1) und (4-1), 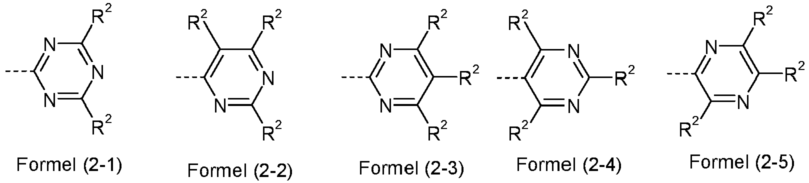 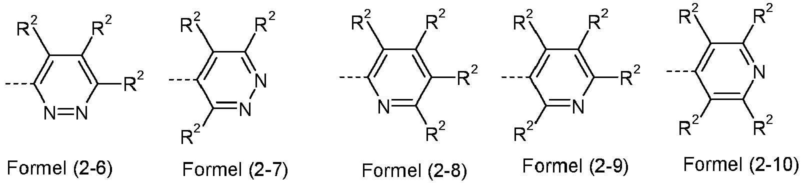 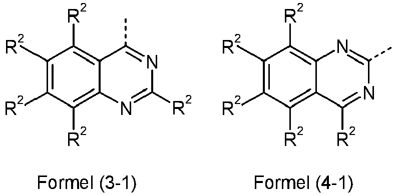 wobei die gestrichelte Bindung die Verknüpfung dieser Gruppen darstellt und R² die oben genannten Bedeutungen aufweist.

Bevorzugt sind die Gruppen der Formeln (2-1) bis (2-3), (3-1) und (4-1), und besonders bevorzugt ist die Gruppe der Formel (2-1).

Bevorzugte Ausführungsformen der oben genannten Gruppen sind die Gruppen der folgenden Formeln (2-1a) bis (4-1a), 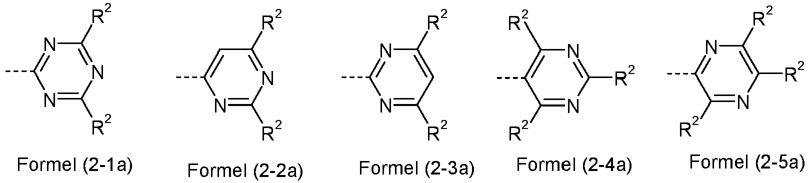 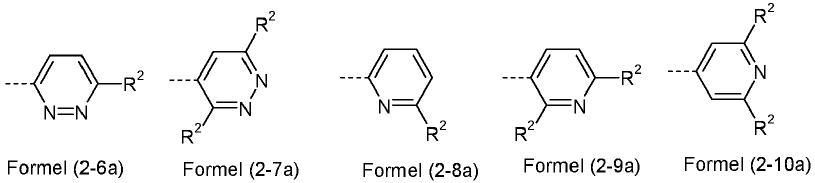 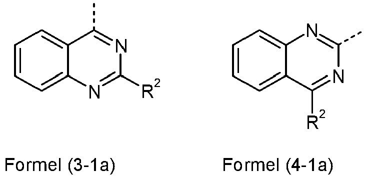 wobei die gestrichelte Bindung die Verknüpfung dieser Gruppen darstellt und R² einen Substituenten gemäß der oben genannten Definition ungleich Wasserstoff darstellt.

Der Substituent R² an der Gruppe HetAr ist bevorzugt gleich oder verschieden bei jedem Auftreten H oder ein aromatisches oder heteroaromatisches Ringsystem mit 6 bis 24 aromatischen Ringatomen, das durch einen oder mehrere Reste R⁴ substituiert sein kann. Dabei ist R² in den Gruppen der Formeln (2-1a) bis (4-1a) ungleich Wasserstoff. Das aromatische oder heteroaromatische Ringsystem hat bevorzugt 6 bis 18 aromatische Ringatome. Besonders bevorzugt handelt es sich um ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R⁴ substituiert sein kann, bevorzugt aber unsubstituiert ist. Beispiele für geeignete Gruppen R² sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl,1-, 2-, 3- oder 4-Fluorenyl, 1-, 2-, 3-oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R⁴ substituiert sein können, bevorzugt aber unsubstituiert sind.

Beispiele für geeignete Gruppen R² sind die im Folgenden aufgeführten Strukturen R²-1 bis R²-29, 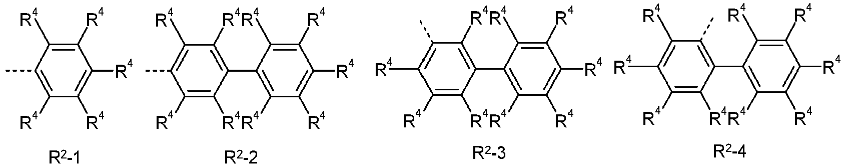 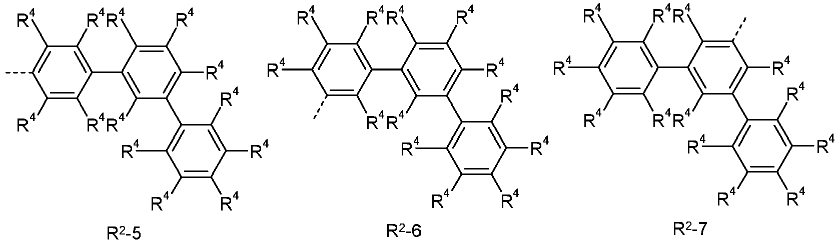 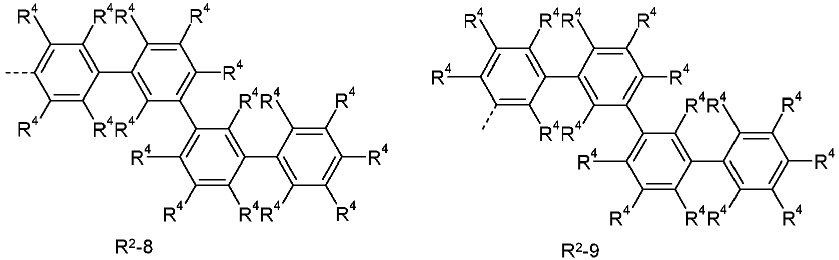 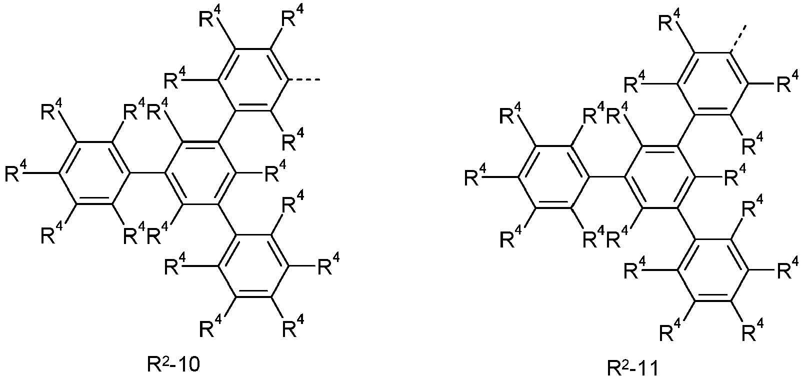 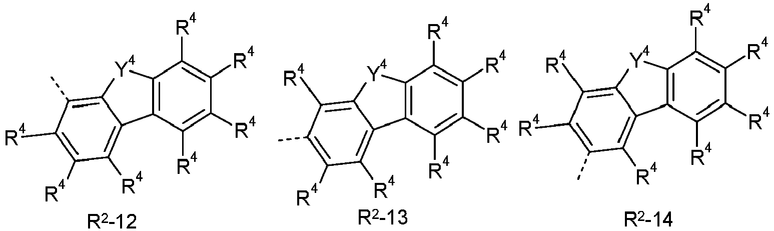 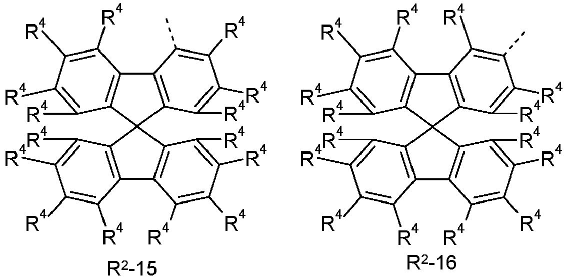 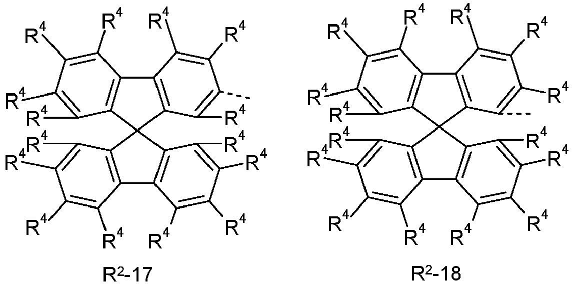 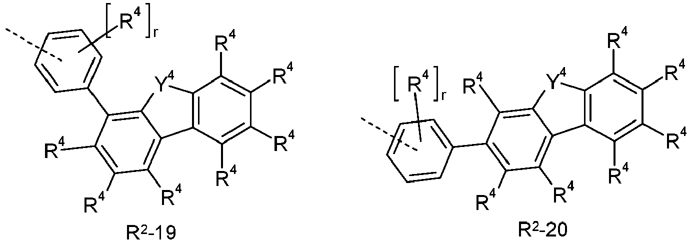 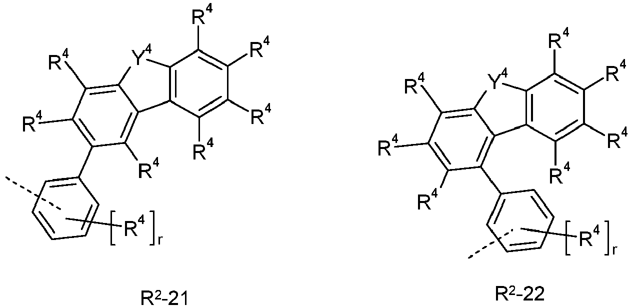 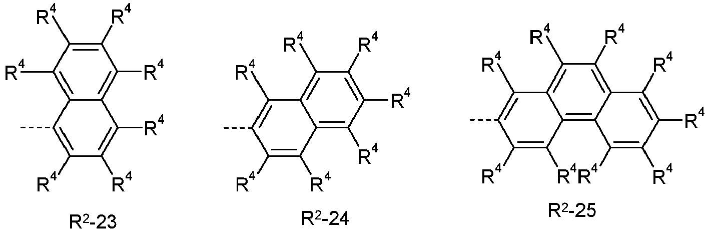 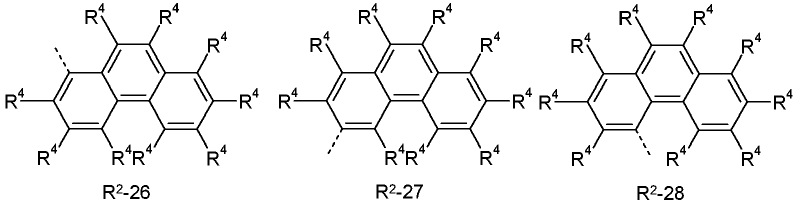 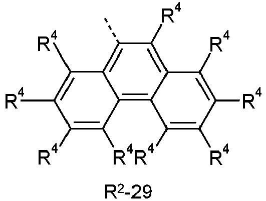 wobei R⁴ die oben genannten Bedeutungen aufweist und die gestrichelte Bindung die Bindung an die Heteroarylgruppe darstellt und r für 0, 1, 2, 3 oder 4 steht und zusätzlich gilt:
- Y⁴: ist bei jedem Auftreten gleich oder verschieden gewählt aus C(R⁴)₂, C=O, O, S, S=O, SO₂, Si(R⁴)₂, NR⁴, wobei der Rest R⁴, der an N gebunden ist, ungleich H oder D ist.

Im Folgenden werden bevorzugte Ausführungsformen der Gruppe N' ausgeführt. Wie oben beschrieben, steht die Gruppe N' für eine Gruppe der Formel (L-1) oder bevorzugt der Formel (L-2) bis (L-7).

In einer bevorzugten Ausführungsform der Erfindung steht in der Gruppe der Formel (L-1) oder den bevorzugten Ausführungsformen die Gruppe Ar gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R³ substituiert sein kann, bevorzugt aber unsubstituiert ist. Beispiele für geeignete Gruppen Ar sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3-oder 4-Fluorenyl, 1-, 2-, 3-oder 4-Spirobifluorenyl, Pyridyl, Pyrimidinyl, 1-, 2-, 3-oder 4-Dibenzofuranyl, 1-, 2-, 3-oder 4-Dibenzothienyl und 1-, 2-, 3- oder 4-Carbazolyl, die jeweils durch einen oder mehrere Reste R³ substituiert sein können, bevorzugt aber unsubstituiert sind.

Besonders bevorzugte Gruppen Ar sind die Gruppen der folgenden Formeln (Ar-1) bis (Ar-29), 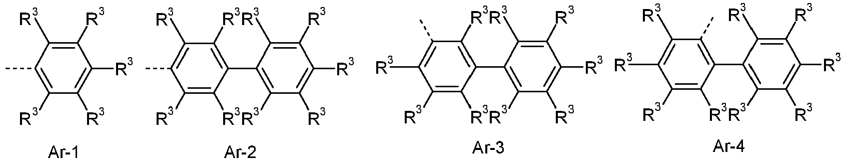 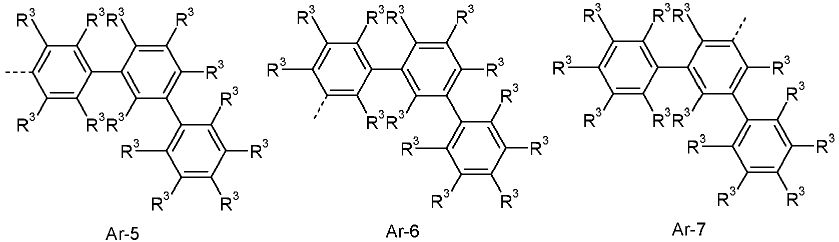 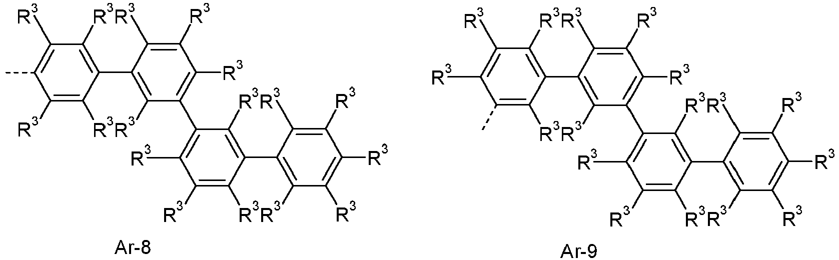 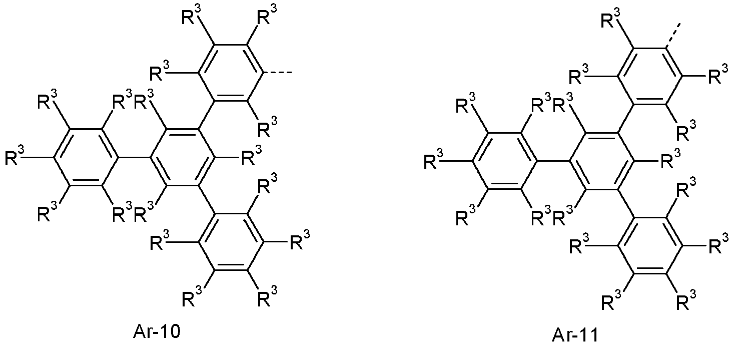 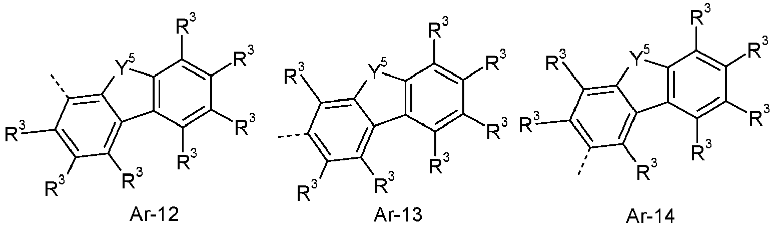 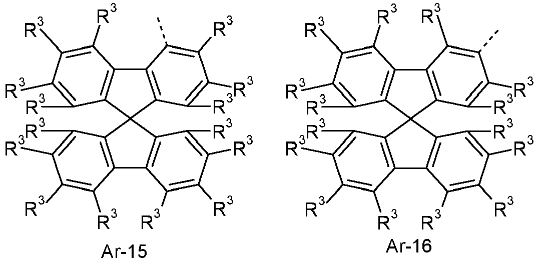 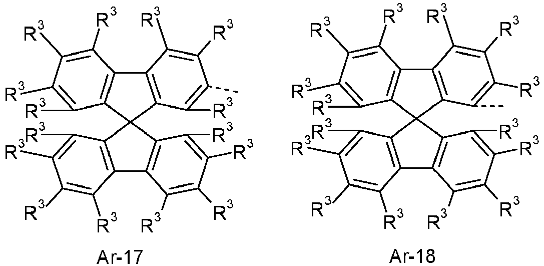 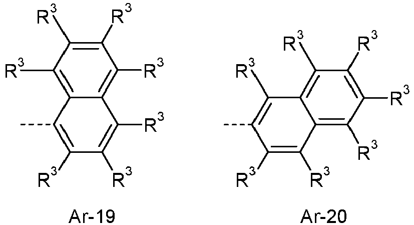 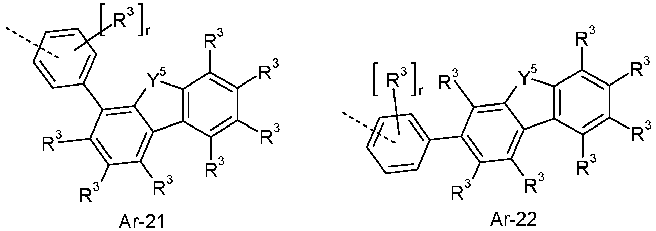 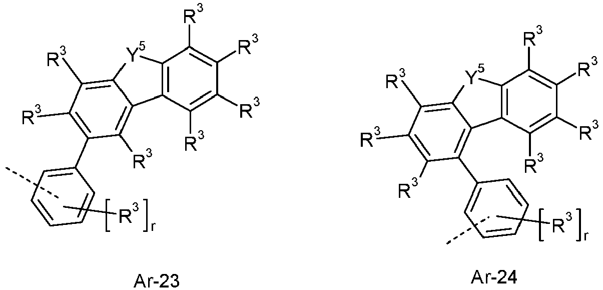 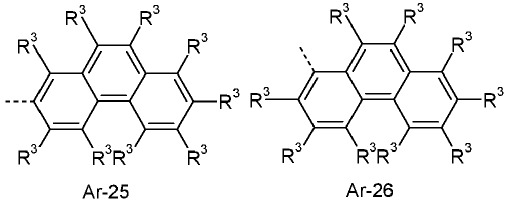 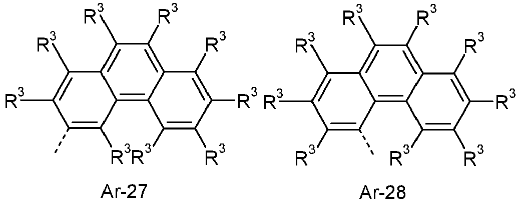 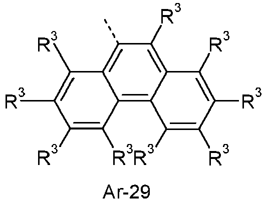 wobei R³ und r die oben genannten Bedeutungen aufweisen und die gestrichelte Bindung die Bindung an den Stickstoff darstellt und an Stelle von R³ soweit erforderlich eine Bindung zu L², Y² oder Y³ stehen kann und zusätzlich gilt:
- Y⁵: ist bei jedem Auftreten gleich oder verschieden gewählt aus C(R³)₂, C=O, O, S, S=O, SO₂, Si(R³)₂, NR³, wobei der Rest R³, der an N gebunden ist, ungleich H oder D ist.

In einer bevorzugten Ausführungsform der Erfindung steht R³, welches nicht an ein Stickstoffatom gebunden ist, gleich oder verschieden bei jedem Auftreten für H, eine Alkylgruppe mit 1 bis 4 C-Atomen oder ein aromatisches oder heteroaromatisches Ringsystem mit 5 bis 14 aromatischen Ringatomen. Besonders bevorzugt steht R³ gleich oder verschieden bei jedem Auftreten für H oder eine Alkylgruppe mit 1 bis 4 C-Atomen.

In einer weiteren bevorzugten Ausführungsform der Erfindung steht Y¹ gleich oder verschieden bei jedem Auftreten für O oder S, besonders bevorzugt für O.

In einer weiteren bevorzugten Ausführungsform der Erfindung stehen Y² und Y³ gleich oder verschieden bei jedem Auftreten für O oder S.

Wenn Y² bzw. Y³, Y⁴ oder Y⁵ für NR, bzw. NR³ oder NR⁴ steht, ist es bevorzugt, wenn dieser Rest R, R³ oder R⁴ bei jedem Auftreten gleich oder verschieden für ein aromatisches oder heteroaromatisches Ringsystem mit 5 bis 24 aromatischen Ringatomen steht, das jeweils mit einem oder mehreren Resten R⁴ bzw. R⁵ substituiert sein kann, besonders bevorzugt für ein aromatisches oder heteroaromatisches Ringsystem mit 6 bis 18 aromatischen Ringatomen stehen, das mit einem oder mehreren Resten R⁴ bzw. R⁵ substituiert sein kann. Beispiele für geeignete Substituenten R, R³ oder R⁴ sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3-oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R⁴ bzw. R⁵ ungleich H oder D substituiert ist. Dabei können diese Gruppen jeweils durch einen oder mehrere Reste R⁵ substituiert sein, sind bevorzugt aber unsubstituiert. Dabei sind geeignete Strukturen R, R³ oder R⁴ die gleichen Strukturen, wie sie vorne für R²-1 bis R²-29 abgebildet sind, wobei die Reste R⁴ im Falle von R⁴ für R⁵ stehen.

Wenn Y² bzw. Y³, Y⁴ oder Y⁵ für C(R)₂, bzw. C(R³)₂ oder C(R⁴)₂, steht, ist es bevorzugt, wenn diese Reste R, R³ oder R⁴ bei jedem Auftreten gleich oder verschieden für eine geradkettige Alkylgruppe mit 1 bis 10 C-Atomen oder eine verzweigte oder cyclische Alkylgruppe mit 3 bis 10 C-Atomen oder eine Alkenylgruppe mit 2 bis 10 C-Atomen stehen, die jeweils mit einem oder mehreren Resten R⁴ oder R⁵ substituiert sein kann, wobei eine oder mehrere nicht-benachbarte CH₂-Gruppen durch O ersetzt sein können und wobei ein oder mehrere H-Atome durch D oder F ersetzt sein können, oder für ein aromatisches oder heteroaromatisches Ringsystem mit 5 bis 24 aromatischen Ringatomen, das jeweils mit einem oder mehreren Resten R⁴ bzw. R⁵ substituiert sein kann; dabei können optional die beiden Substituenten R, R³ oder R⁴ ein monocyclisches oder polycyclisches, aliphatisches, aromatisches oder heteroaromatisches Ringsystem bilden, das mit einem oder mehreren Resten R⁴ bzw. R⁵ substituiert sein kann. Durch Ringbildung der beiden Substituenten R, R³ oder R⁴ wird ein Spirosystem aufgespannt, beispielsweise ein Spirobifluoren bzw. Derivat eines Spirobifluorens, wenn die Gruppen R, R³ oder R⁴ für Phenylgruppen stehen.

In einer weiteren bevorzugten Ausführungsform der Erfindung steht L¹ für eine Einfachbindung oder ein aromatisches oder heteroaromatisches Ringsystem mit 5 bis 24 aromatischen Ringatomen, das durch einen oder mehrere Reste R³ substituiert sein kann. Besonders bevorzugt steht L¹ für eine Einfachbindung, ein aromatisches Ringsystem mit 6 bis 12 aromatischen Ringatomen oder ein heteroaromatisches Ringsystem mit 6 bis 13 aromatischen Ringatomen, das jeweils durch einen oder mehrere Reste R³ substituiert sein kann, bevorzugt aber unsubstituiert ist. Besonders bevorzugt steht L¹ für eine Einfachbindung oder ein aromatisches Ringsystem mit 6 bis 12 aromatischen Ringatomen, insbesondere für eine Einfachbindung. Beispiele für geeignete aromatische oder heteroaromatische Ringsysteme L¹ sind ausgewählt aus der Gruppe bestehend aus Phenylen, Biphenyl, Fluoren, Pyridin, Pyrimidin, Triazin, Dibenzofuran, Dibenzothiophen und Carbazol, die jeweils durch einen oder mehrere Reste R³ substituiert sein können, bevorzugt aber unsubstituiert sind.

In einer weiteren bevorzugten Ausführungsform der Erfindung steht L² für eine Einfachbindung oder ein aromatisches oder heteroaromatisches Ringsystem mit 5 bis 24 aromatischen Ringatomen, das durch einen oder mehrere Reste R³ substituiert sein kann. Besonders bevorzugt steht L² für eine Einfachbindung, ein aromatisches Ringsystem mit 6 bis 12 aromatischen Ringatomen oder ein heteroaromatisches Ringsystem mit 6 bis 13 aromatischen Ringatomen, das jeweils durch einen oder mehrere Reste R³ substituiert sein kann, bevorzugt aber unsubstituiert ist. Ganz besonders bevorzugt steht L² für eine Einfachbindung oder ein aromatisches Ringsystem mit 6 bis 12 aromatischen Ringatomen, insbesondere für eine Einfachbindung. Beispiele für geeignete aromatische oder heteroaromatische Ringsysteme L² sind ausgewählt aus der Gruppe bestehend aus Phenylen, Biphenyl, Fluoren, Pyridin, Pyrimidin, Triazin, Dibenzofuran, Dibenzothiophen und Carbazol, die jeweils durch einen oder mehrere Reste R³ substituiert sein können, bevorzugt aber unsubstituiert sind, insbesondere ausgewählt aus der Gruppe bestehend aus Phenylen, Biphenyl, Fluoren, Pyridin, Pyrimidin, Triazin, Dibenzofuran und Dibenzothiophen.

Wenn die erfindungsgemäßen Verbindungen Substituenten R, R¹, R² oder R³ aufweisen, dann sind diese bevorzugt gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁴ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁴ substituiert sein kann, oder einer Aralkyl- oder Heteroaralkylgruppe mit 5 bis 25 aromatischen Ringatomen, die mit einem oder mehreren Resten R⁴ substituiert sein kann; dabei können optional zwei Substituenten R, bzw. zwei Substituenten R¹, bzw. zwei Substituenten R², bzw. zwei Substituenten R³, die an benachbarte Kohlenstoffatome gebunden sind, ein monocyclisches oder polycyclisches, aliphatisches Ringsystem bilden, das mit einem oder mehreren Resten R⁴ substituiert sein kann.

Wenn die erfindungsgemäßen Verbindungen Substituenten R⁴ aufweisen, dann sind diese bevorzugt gewählt aus der Gruppe bestehend aus H, D, F, CN, N(R⁵)₂, C(=O)R⁵, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁵ substituiert sein kann, oder einer Aralkyl- oder Heteroaralkylgruppe mit 5 bis 25 aromatischen Ringatomen, die mit einem oder mehreren Resten R⁵ substituiert sein kann; dabei können optional zwei Substituenten R⁴, die an benachbarte Kohlenstoffatome gebunden sind, ein monocyclisches oder polycyclisches, aliphatisches Ringsystem bilden, das mit einem oder mehreren Resten R⁵ substituiert sein kann.

Ganz besonders bevorzugt sind die Substituenten R, R¹, R² oder R³ ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R⁴ substituiert sein kann, bevorzugt aber unsubstituiert ist. Beispiele für geeignete Substituenten R, R¹, R² oder R³ sind ausgewählt aus der Gruppe bestehend aus Phenyl, Biphenyl, insbesondere ortho-, meta- oder para-Biphenyl, Terphenyl, insbesondere verzweigtes Terphenyl, Quaterphenyl, insbesondere verzweigtes Quaterphenyl, 1-, 2-, 3-oder 4-Fluorenyl, 1-, 2-, 3-oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R⁴ substituiert sein können, bevorzugt aber unsubstituiert sind. Dabei sind geeignete Strukturen R, R¹, R² oder R³ die gleichen Strukturen, wie sie vorne für R²-1 bis R²-29 abgebildet sind.

In einer weiteren bevorzugten Ausführungsform der Erfindung ist R⁵ bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Wenn die erfindungsgemäße Verbindung mit aromatischen oder heteroaromatischen Gruppen substituiert ist, so ist es bevorzugt, wenn diese keine Aryl- oder Heteroarylgruppen mit mehr als zwei direkt aneinander kondensierten aromatischen Sechsringen aufweisen. Besonders bevorzugt weisen die Substituenten überhaupt keine Aryl- oder Heteroarylgruppen mit direkt aneinander kondensierten Sechsringen auf. Diese Bevorzugung ist mit der geringen Triplettenergie derartiger Strukturen zu begründen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Die oben genannten Bevorzugungen können einzeln oder gemeinsam auftreten. Es ist bevorzugt, wenn die oben genannten Bevorzugungen gemeinsam auftreten.

Bevorzugt sind somit Verbindungen gemäß der oben genannten Formel (1a), für die gilt:
- L¹: ist eine Einfachbindung oder ein aromatisches oder heteroaromatisches Ringsystem mit 5 bis 30 aromatischen Ringatomen, das durch einen oder mehrere Reste R³ substituiert sein kann;
- L²: ist eine Einfachbindung oder ein aromatisches oder heteroaromatisches Ringsystem mit 5 bis 30 aromatischen Ringatomen, das durch einen oder mehrere Reste R³ substituiert sein kann;
- N': ist eine Gruppe der Formeln (L-5), (L-6) oder (L-7);
- HetAr: ist eine Gruppe gemäß einer der oben aufgeführten Formeln (2-1) bis (2-10), (3-1) oder (4-1);
- Ar: ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann;
- Y¹: ist gleich oder verschieden bei jedem Auftreten O oder S;
- R, R¹, R², R³: ist bei jedem Auftreten gleich oder verschieden ausgewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁴ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁴ substituiert sein kann, oder einer Aralkyl- oder Heteroaralkylgruppe mit 5 bis 25 aromatischen Ringatomen, die mit einem oder mehreren Resten R⁴ substituiert sein kann; dabei können optional zwei Substituenten R, bzw zwei Substituenten R¹, bzw. zwei Substituenten R², bzw. zwei Substituenten R³, die an benachbarte Kohlenstoffatome gebunden sind, ein monocyclisches oder polycyclisches, aliphatisches Ringsystem bilden, das mit einem oder mehreren Resten R⁴ substituiert sein kann.
- R⁴: ist bei jedem Auftreten gleich oder verschieden ausgewählt aus der Gruppe bestehend aus H, D, F, CN, N(R⁵)₂, C(=O)R⁵, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R⁵ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R⁵ substituiert sein kann, oder einer Aralkyl- oder Heteroaralkylgruppe mit 5 bis 25 aromatischen Ringatomen, die mit einem oder mehreren Resten R⁵ substituiert sein kann; dabei können optional zwei Substituenten R⁴, die an benachbarte Kohlenstoffatome gebunden sind, ein monocyclisches oder polycyclisches, aliphatisches Ringsystem bilden, das mit einem oder mehreren Resten R⁵ substituiert sein kann;
die weiteren Symbole haben die oben genannten Bedeutungen.

Beispiele für geeignete erfindungsgemäße Verbindungen sind die nachstehend gezeigten Strukturen.

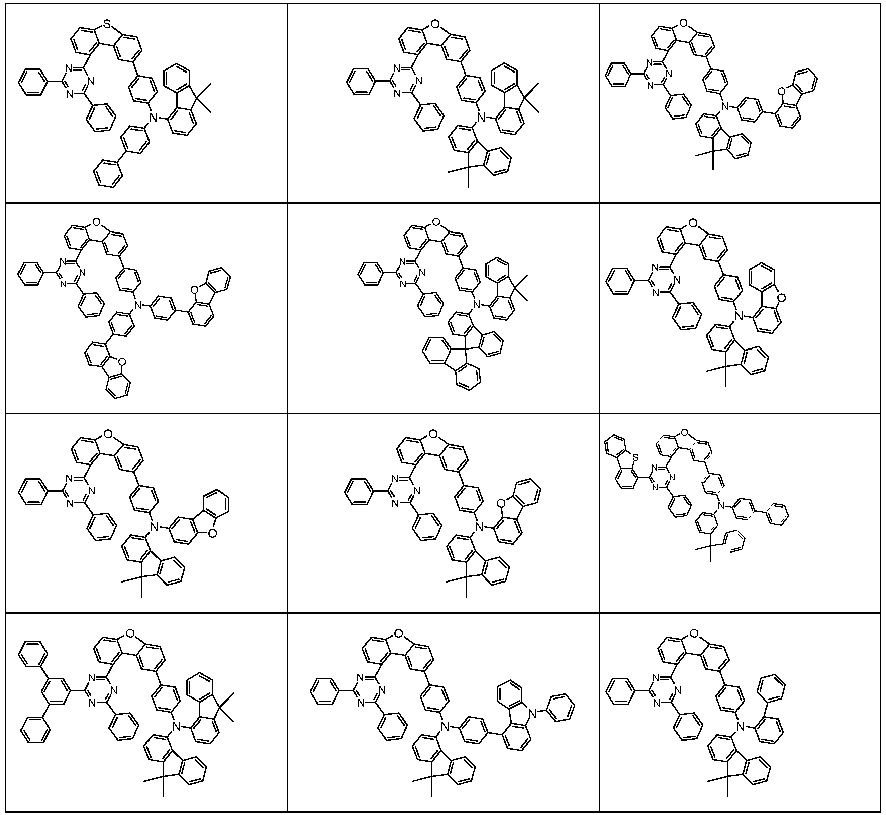

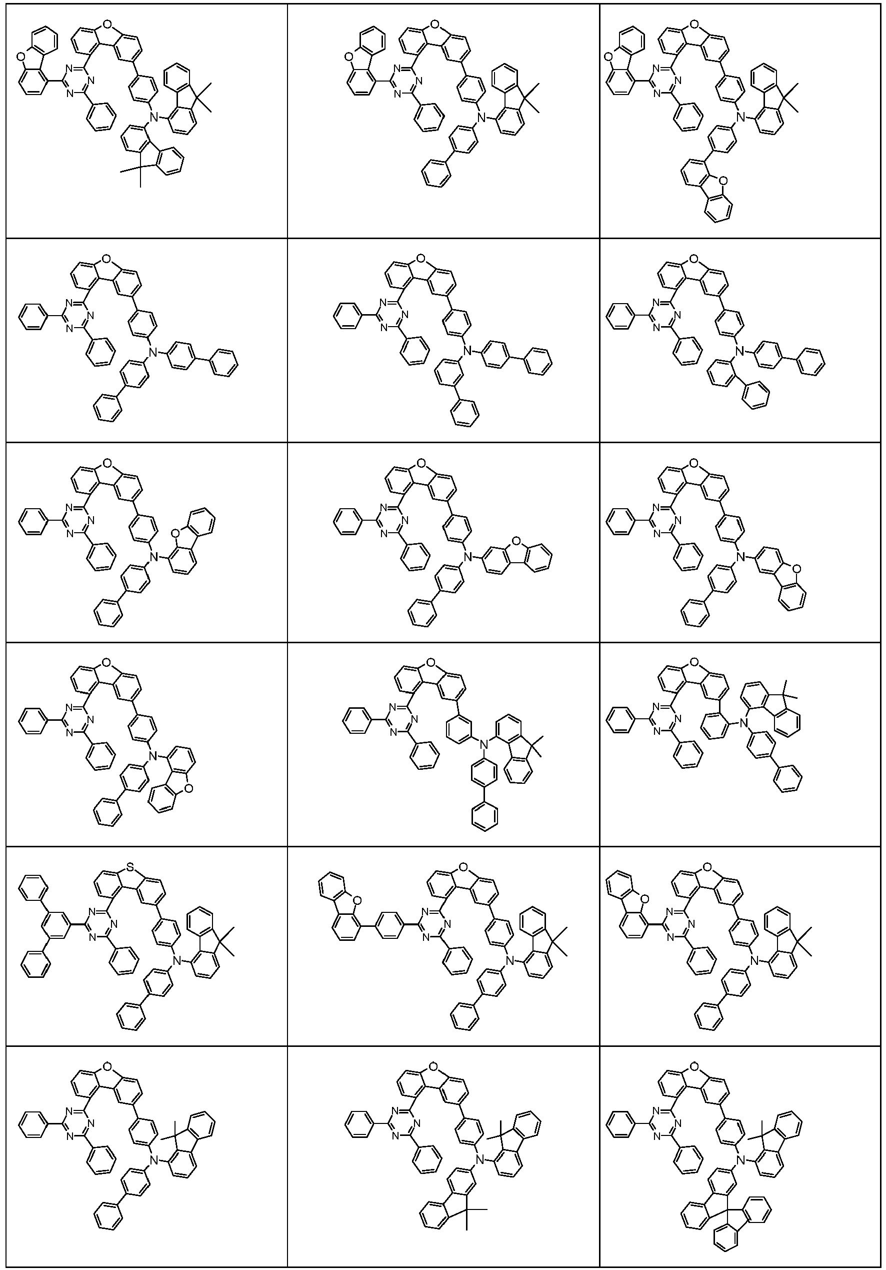

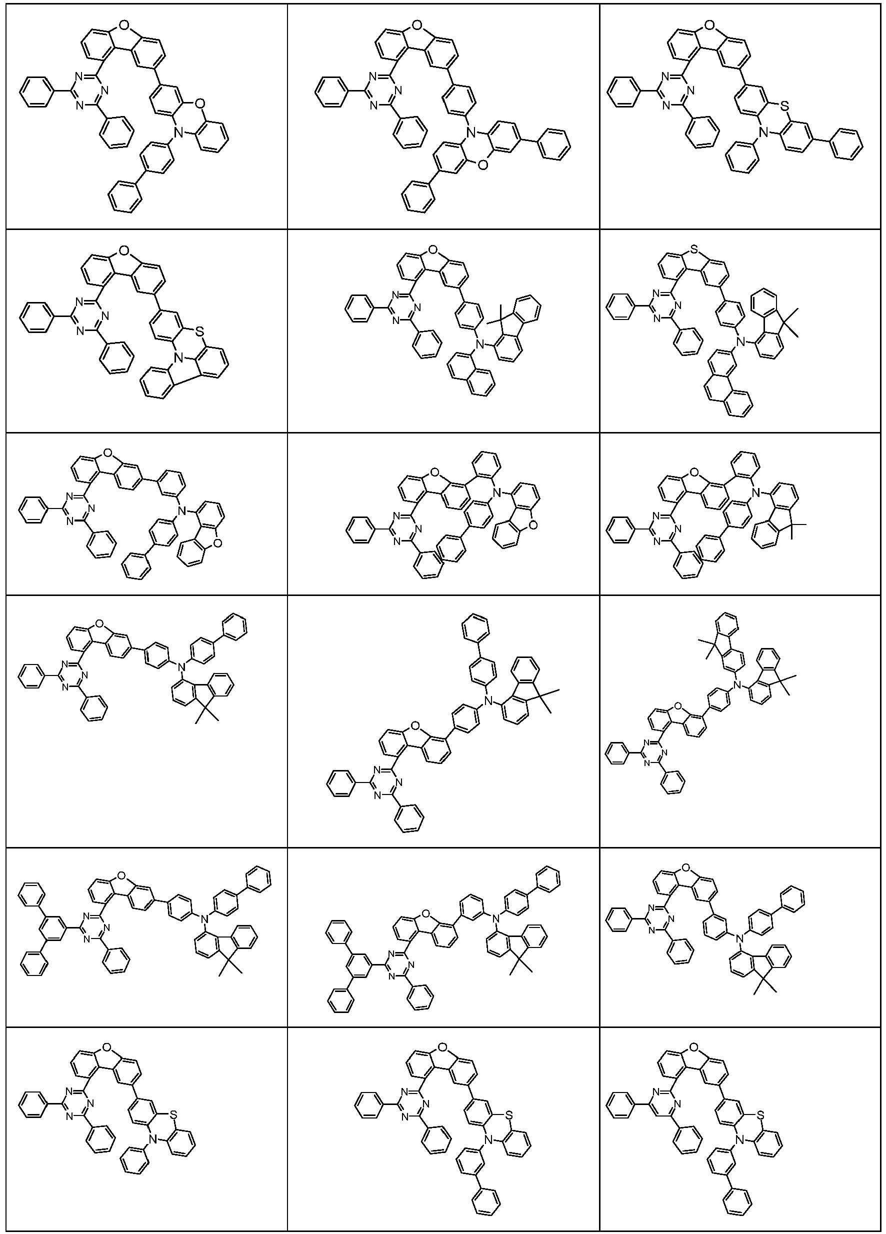

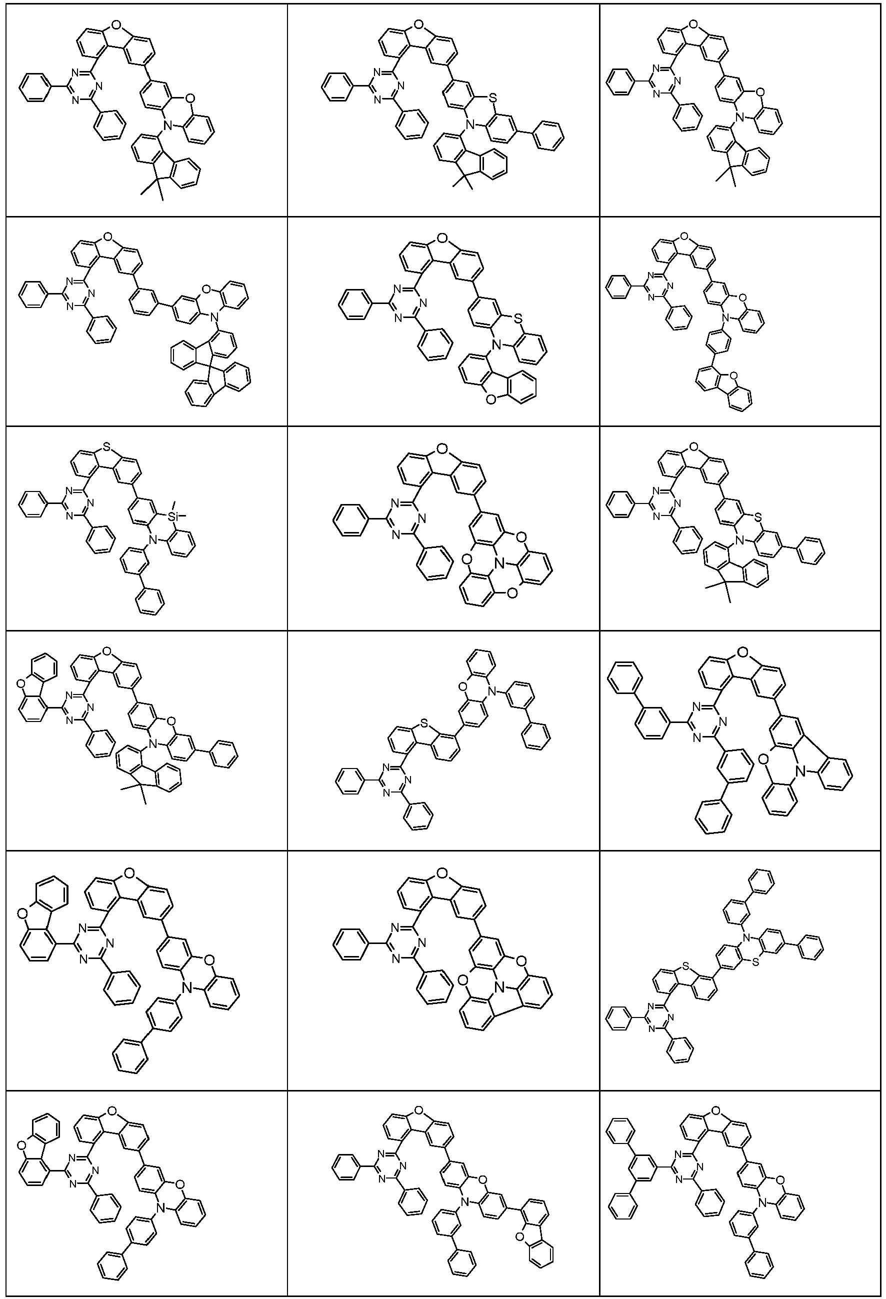

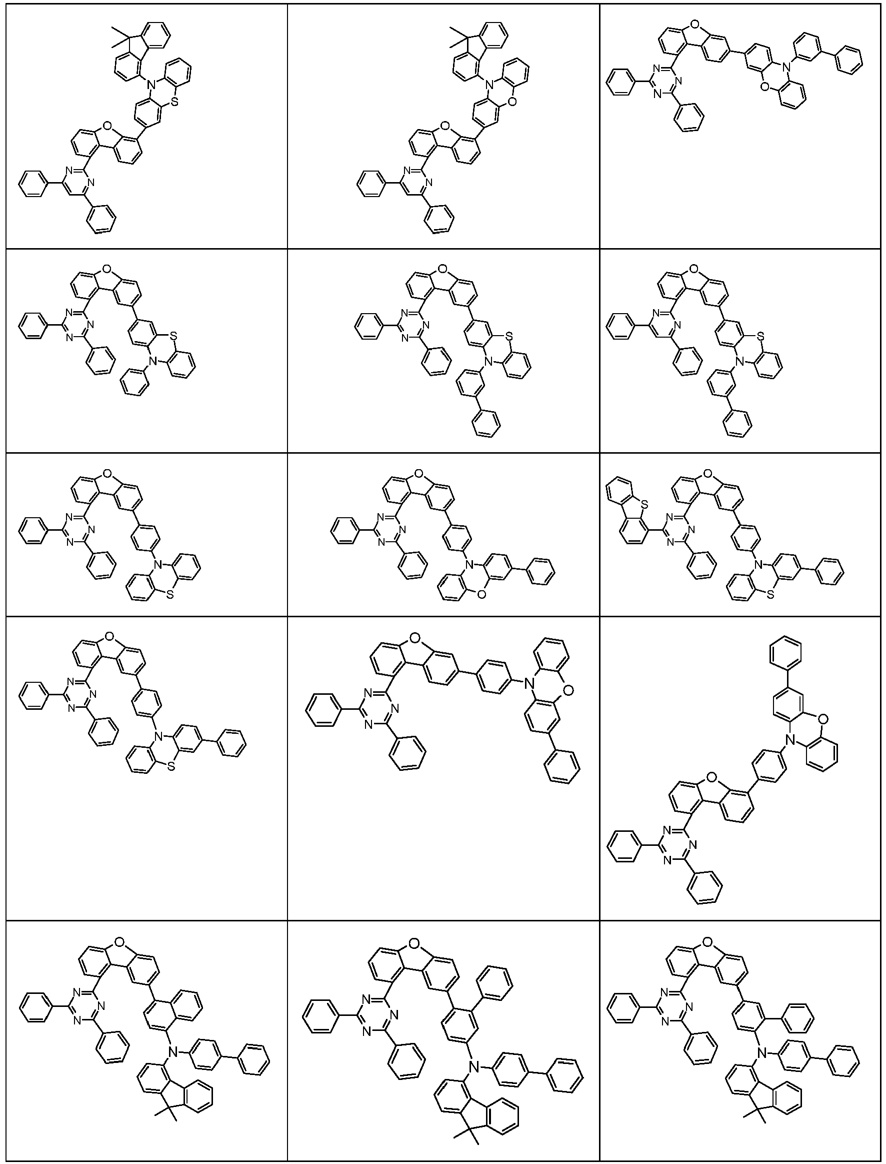

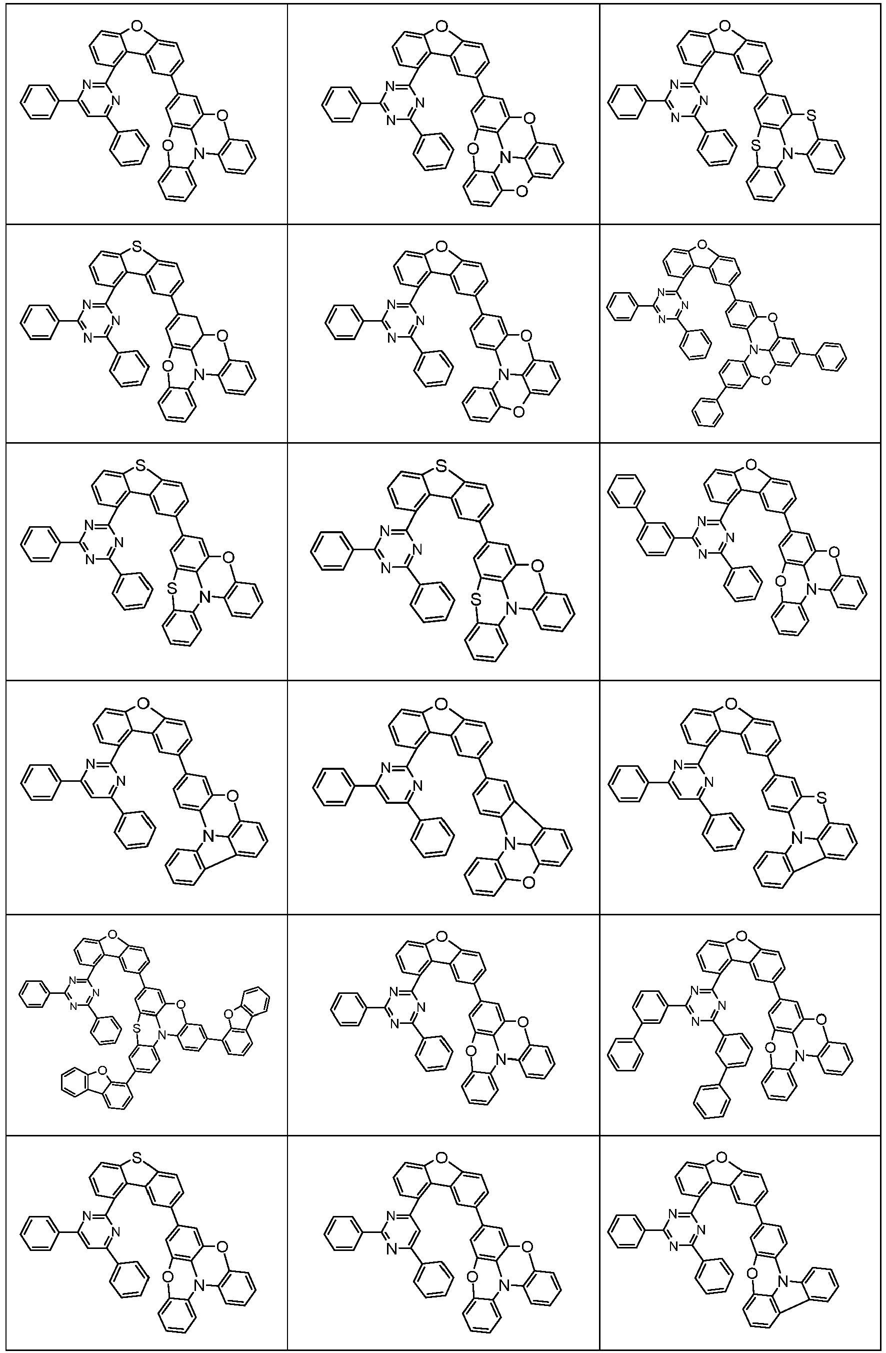

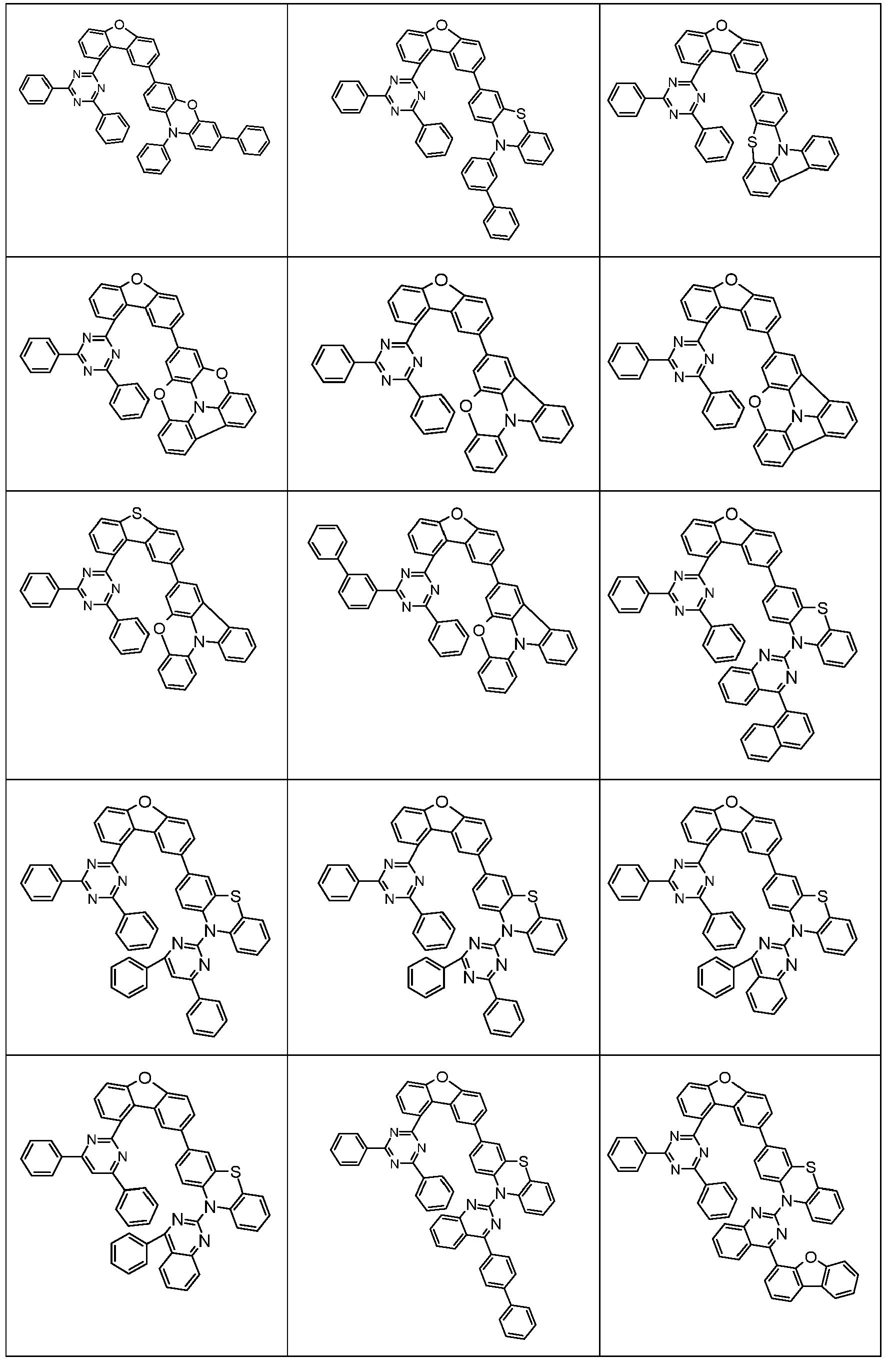

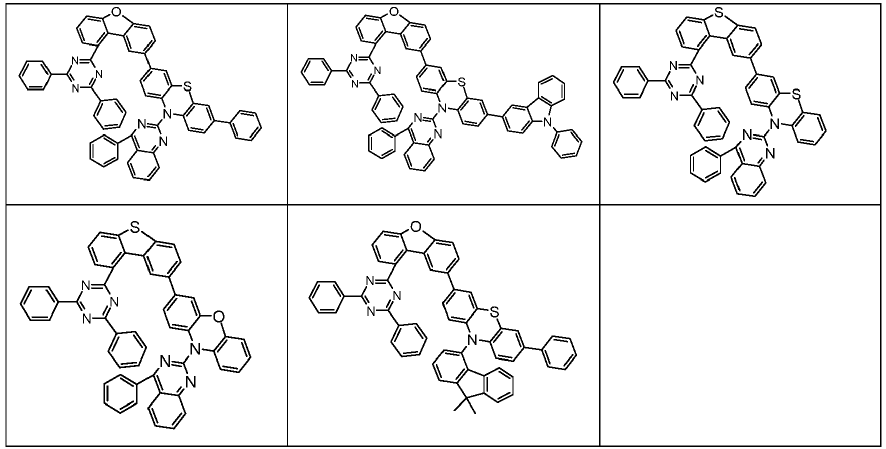

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden.

Geignete Syntheseverfahren sind allgemein in den folgenden Schemata 1 bis 5 dargestellt.

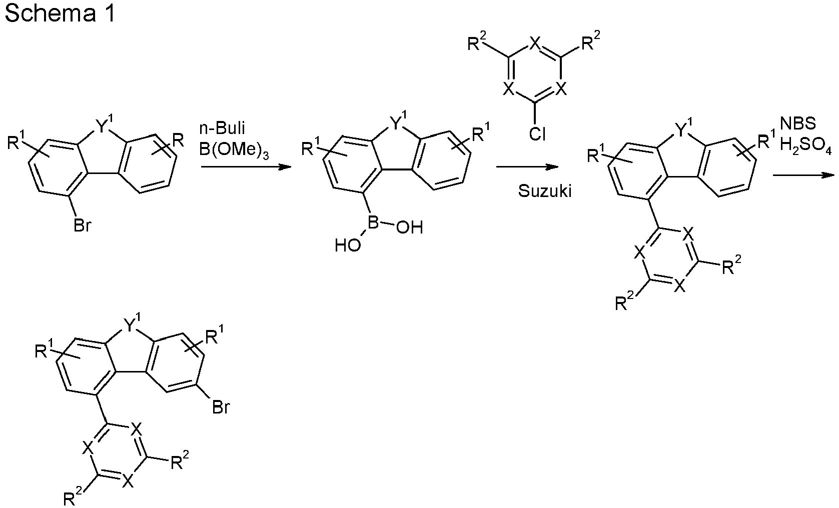

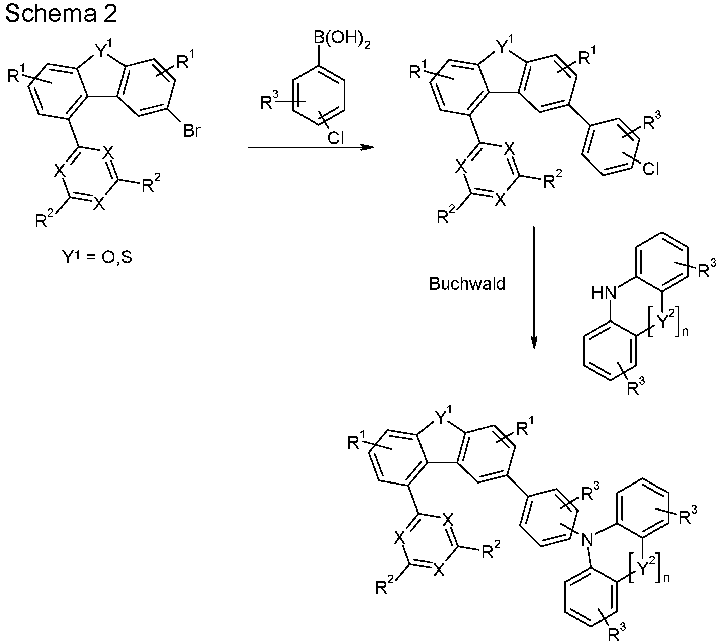

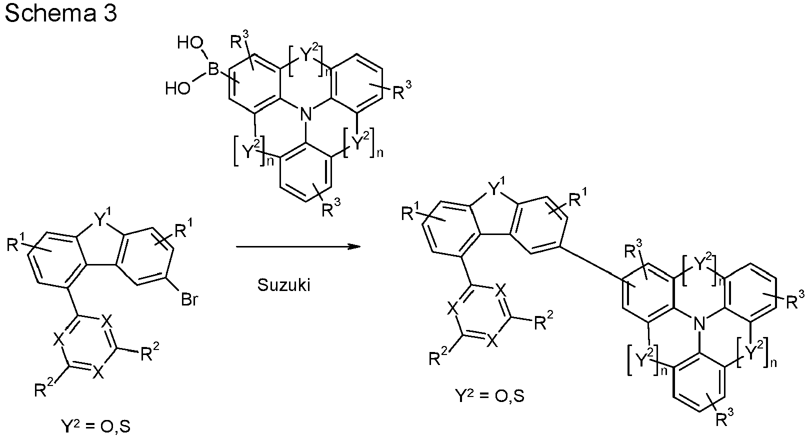

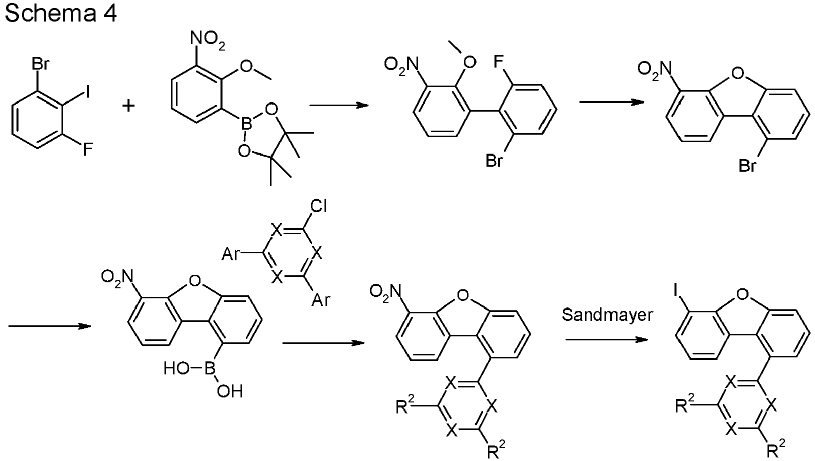

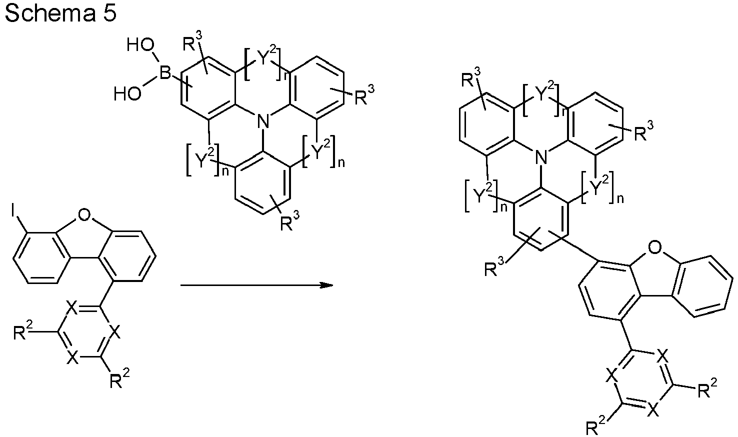

Die Synthese geht von 1-Halogen-dibenzofuran bzw. -dibenzothiophen aus, welches zur entsprechenden Boronsäure bzw. einem Boronsäurederivat umgesetzt wird. Im nächsten Schritt kann die Gruppe HetAr durch Suzuki-Kupplung eingeführt werden. Analog kann auch eine Gruppe L¹-HetAr eingeführt werden. Die Halogenierung, beispielsweise mit NBS, erfolgt selektiv in 8-Position des Dibenzofurans bzw. Dibenzothiophens. Im letzten Schritt kann in dieser Position, beispielsweise durch eine Hartwig-Buchwald-Kupplung oder eine Suzuki-Kupplung, die Gruppe N' eingeführt werden.

Schemata 4 und 5 zeigen ein alternatives Syntheseverfahren, bei dem das Dibenzofuran aufgebaut wird, um die Gruppe N' an der 1-Position einführen zu können.

Die gezeigten allgemeinen Verfahren zur Synthese der erfindungsgemäßen Verbindungen sind exemplarisch. Der Fachmann kann alternative Synthesewege im Rahmen seines allgemeinen Fachwissens entwickeln.

Ein weiterer Gegenstand der vorliegenden Erfindung ist ein Verfahren zur Synthese der erfindungsgemäßen Verbindungen, ausgehend von 1-Halogen-dibenzofuran bzw. 1-Halogen-dibenzothiophen, wobei das Halogen bevorzugt Brom ist, gekennzeichnet durch die folgenden Schritte:
(1) optional Umsetzung der Halogengruppe zu einer Boronsäure bzw. einem Boronsäurederivat;
(2) Einführung der Gruppe HetAr bzw. L¹-HetAr durch eine Kupplungsreaktion, insbesondere eine Suzuki-Kupplung;
(3) Halogenierung, insbesondere Bromierung, des Dibenzofurans bzw. Dibenzothiophens in 8-Position;
(4) Einführung der Gruppe N' durch eine Kupplungsreaktion, insbesondere eine Hartwig-Buchwald-Kupplung oder eine Suzuki-Kupplung.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthyl-isovalerat, Cyclohexylhexanoat oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Weiterhin bevorzugt sind Tandem-OLEDs. Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter oder für Emitter, die TADF (thermally activated delayed fluorescence) zeigen, insbesondere für phosphoreszierende Emitter, und/oder in einer Elektronentransportschicht und/oder in einer elektronenblockierenden bzw. exzitonenblockierenden Schicht und/oder in einer Lochtransportschicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält. Die erfindungsgemäßen Verbindungen zeichnen sich insbesondere dadurch aus, dass sie vorteilhafte Eigenschaften auch mit phosphoreszierenden Emittern, welche Ketoketonatliganden enthalten, aufweisen.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand, verstanden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. gemäß den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 und WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, überbrückte Carbazol-Derivate, z. B. gemäß US 2009/0136779, WO 2010/050778, WO 2011/042107, WO 2011/088877 oder WO 2012/143080, Triphenylenderivaten, z. B. gemäß WO 2012/048781, Dibenzofuranderivate, z. B. gemäß WO 2017/148564 oder WO 2017/148565, oder Lactame, z. B. gemäß WO 2011/116865 oder WO 2011/137951. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Lactame, Carbazolderivate und Indenocarbazolderivate.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094962, WO 2014/094961, WO 2014/094960, WO 2015/036074, WO 2015/104045, WO 2015/117718, WO 2016/015815, WO 2016/124304, WO 2017/032439 und der noch nicht offen gelegten Anmeldung EP 16179378.1 entnommen werden entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen. Diese können bevorzugt auch mit einer oder mehreren weiteren elektronentransportierenden Gruppen substituiert sein, beispielsweise Benzimidazolgruppen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen verwenden.

Weiterhin bevorzugt ist eine organische Elektrolumineszenzvorrichtung, dadurch gekennzeichnet, dass eine oder mehrere Schichten mit einem Sublimationsverfahren aufgebracht werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (OrganicVapour Phase Deposition) Verfahren oder mit Hilfe einer Trägergassublimation aufgebracht werden. Dabei werden die Materialien bei einem Druck zwischen 10⁻⁵ mbar und 1 bar aufgebracht. Ein Spezialfall dieses Verfahrens ist das OVJP (OrganicVapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar. Weiterhin weisen die Verbindungen eine hohe Glasübergangstemperatur und eine hohe thermische Stabilität auf.

Die Erfindung wird nun durch die nachfolgenden Beispiele näher erläutert, ohne sie dadurch einschränken zu wollen.

### Beispiele

### Synthesebeispiele

Die nachfolgenden Synthesen werden, sofern nicht anders angegeben, unter einer Schutzgasatmosphäre in getrockneten Lösungsmitteln durchgeführt. Die erfindungsgemäßen Verbindungen können mittels dem Fachmann bekannten Syntheseverfahren dargestellt werden.

### a) Triazinsynthese:

### 2,4-Bis-biphenyl-3-yl-6-chlor-[1,3,5]triazin

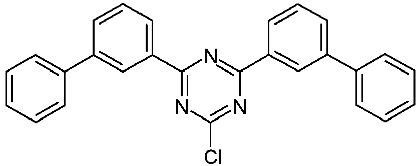

5.2 g Magnesium (0.215 mol) werden in einem 500 mL Vierhalskolben vorgelegt, und eine Lösung aus 50 g 3-Brombiphenyl (214 mmol) in 200 ml THF wird langsam zugetropft. Die Reaktionsmischung wird 1.5 h zum Sieden erhitzt und anschließend auf Raumtemperatur abgekühlt. In einem zweiten Kolben wird Cyanchlorid (17.2 g, 93 mmol) in 150 ml THF vorgelegt und auf 0 °C gekühlt. Bei dieser Temperatur wird das abgekühlte Grignard-Reagenz zugetropft und 12 h bei RT gerührt. Nach dieser Zeit wird die Reaktionsmischung mit 150 mL HCl versetzt und die wässrige Phase dreimal mit Dichlormethan extrahiert. Die vereinigten organischen Phasen werden mit Wasser gewaschen, über Na₂SO₄ getrocknet und eingeengt. Der Rückstand wird aus EtOH umkristallisiert. Die Ausbeute beträgt 32.8 g (78 mmol, 84%).

### b) 2-Biphenyl-3-yl-4,6-dichlor-[1,3,5]triazin

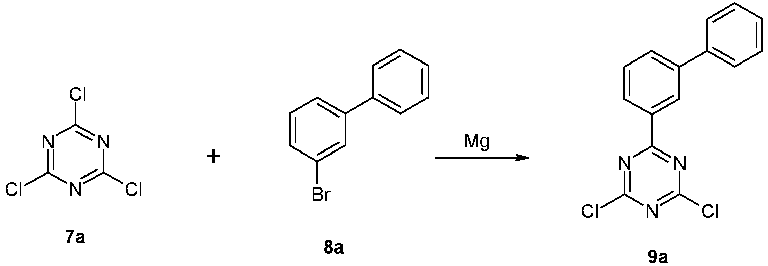

7.9 g (330 mmol, 1.2 eq) Magnesiumspäne werden in einem 1L-Vierhalskolben vorgelegt und so langsam mit einer THF-Lösung aus 63 g (270 mmol, 1.0 eq) 3-Brombiphenyl **8a** (CAS 2113-57-7) versetzt, um den Rückfluss des Reaktionsgemisches zu erhalten. Nach beendeter Zugabe wird der Ansatz für weitere 2 h unter Rückfluss erhitzt.

In einem 2L-Vierhalskolben werden 50 g (270 mmol, 1 eq) 2,4,6-Trichlor-[1,3,5]triazin **7a** (CAS 108-77-0) in 500 ml THF auf -10 °C abgekühlt. Bei dieser Temperatur wird die Grignard-Lösung so langsam zugetropft, dass die Temperatur 0 °C nicht übersteigt, und der Ansatz schließlich über Nacht bei Raumtemperatur gerührt. Zur Aufarbeitung werden 270 ml 1N Salzsäure zugetropft und das Gemisch für 1 h gerührt. Anschließend wird die wässrige Phase abgetrennt und mit Diethylether extrahiert. Die vereinten organischen Phasen werden über Natriumsulfat getrocknet und das Lösungsmittel am Rotationsverdampfer entfernt. Es werden 56 g (69%) eines farblosen Öls **9a** erhalten.

Analog können folgende Verbindungen erhalten werden:

| **Nr.** | **Edukt 7** | **Edukt 8** | **Produkt 9** | **Ausbeute** |
|---|---|---|---|---|
| **1b** | 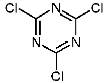 | 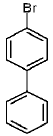 92-66-0 | 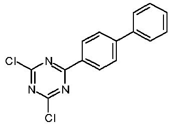 | 56% |
| **2b** | 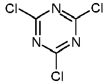 | 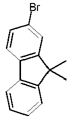 28320-31-2 | 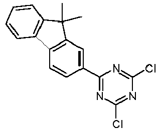 | 27% |
| **3b** | 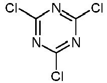 | 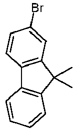 28320-31-2 (Einsatz von 2 eq.) | 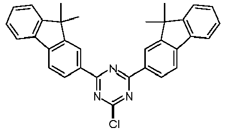 | 48% |
| **4b** | 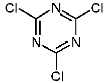 | 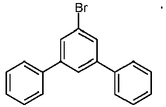 103068-20-8 | 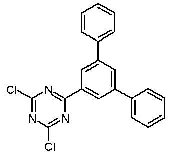 | 71% |

### c) 2-Biphenyl-3-yl-4-chlor-6-(9,9'-spirobi-9H-fluoren-2-yl)-[1,3,5]triazin

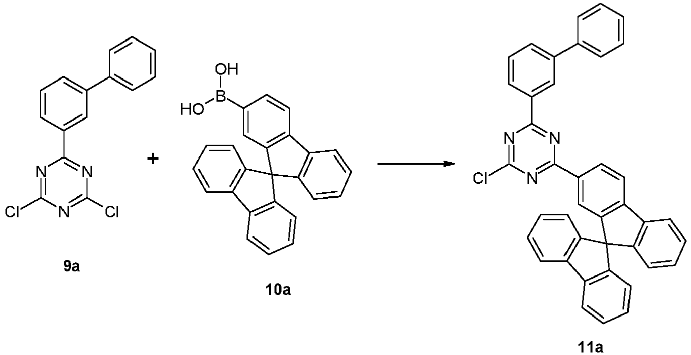

### Variante A:

18 g (50 mmol, 1 eq.) 9,9-Spirobifluoren-2-yl-boronsäure **10a** (CAS 236389-21-2) werden zusammen mit 15 g (50 mmol, 1 eq.) 2-Biphenyl-3-yl-4,6-dichlor-[1,3,5]-triazin **9a** und 5.8 g (55 mmol, 1.1 eq.) Natriumcarbonat in einem Gemisch aus 200 ml Dioxan, 200 ml Toluol und 70 ml Wasser gelöst und für 30 Minuten entgast. Anschließend werden 580 mg (0.50 mmol, 1 mol-%) Tetrakis(triphenylphosphin)palladium (CAS 14221-01-3) zugegeben und der Ansatz über Nacht am Rückfluss erhitzt.

Das Reaktionsgemisch wird abgekühlt und mit 300 ml Wasser versetzt. Die wässrige Phase wird dreimal mit Ethylacetat extrahiert, die organischen Phasen vereint und das Lösungsmittel am Rotationsverdampfer entfernt. Nach Heißextraktion mit Heptan/Toluol 4:1 werden 15 g (26 mmol, 51%) eines farblosen Feststoffs erhalten.

### Variante B: analog b)

Analog können folgende Verbindungen erhalten werden:

| Nr. | **Variante** | **Edukt 9** | **Edukt 10** | **Produkt 11** | **Ausbeute** |
|---|---|---|---|---|---|
| **1c** | B | 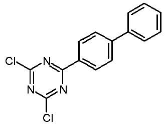 | 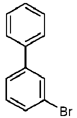 2113-57-7 | 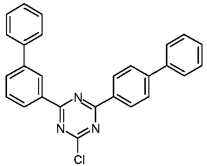 | 68% |
| **2c** | A | 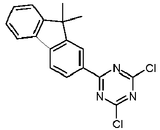 | 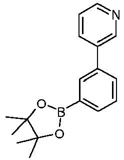 939430-30-5 | 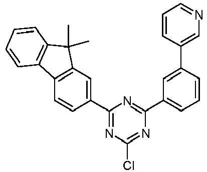 | 81% |
| **3c** | A | 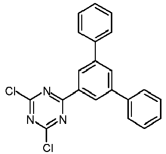 | 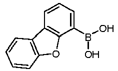 100124-06-9 | 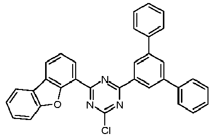 | 63% |
| **4c** | A | 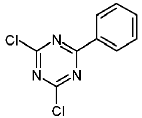 | 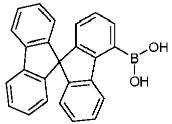 1421789-05-0 | 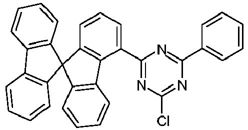 | 71% |
| **5c** | A | 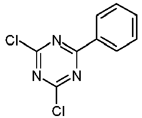 | 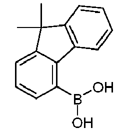 1246022-50-3 | 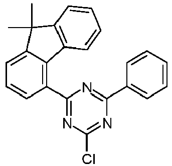 | 53% |
| **8c** | A | 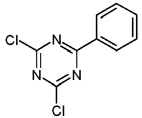 | 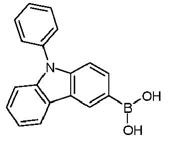 854952-58-2 | 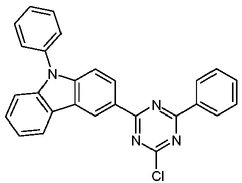 | 68% |
| **9c** | B | 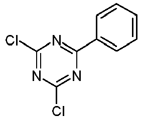 | 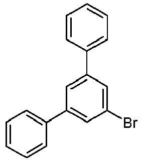 103068-20-8 | 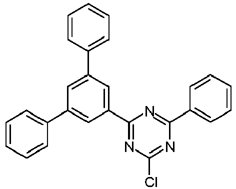 | 67% |

### d) 6-Brom-2-fluor-2'-methoxy-biphenyl

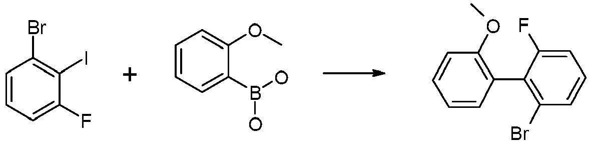

200 g (664 mmol) 1-Brom-3-fluor-2-iod-benzol und 101 g (664 mmol) 2-Methoxyphenylboronsäure und 137.5 g (997 mmol) Natriumtetraborat werden in 1000 mL THF und 600 ml Wasser gelöst und entgast. Es wird mit 9.3 g (13.3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

Analog können folgende Verbindungen erhalten werden:

| | **Edukt 1** | **Edukt 2** | **Produkt** | **Ausbeute** |
|---|---|---|---|---|
| d1 | 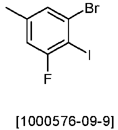 | 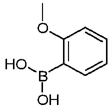 | 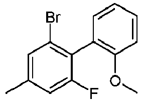 | 77% |
| d2 | 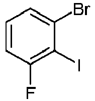 | 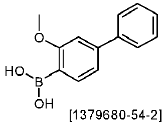 | 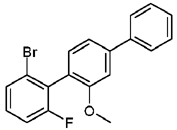 | 74% |
| d3 | 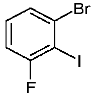 | 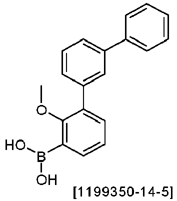 | 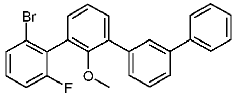 | 76% |
| d4 | 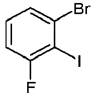 | 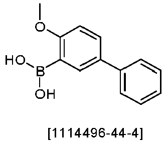 | 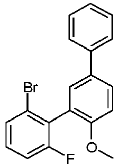 | 71% |

### e) 6'-Brom-2'-fluor-biphenyl-2-ol

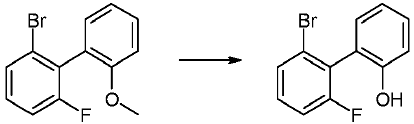

112 g (418 mmol) 6-Brom-2-fluor-2'-methoxy-biphenyl werden in 2 L Dichlormethan gelöst und auf 5 °C gekühlt. Zu dieser Lösung wird innerhalb von 90 min. 41.01 ml (431 mmol) Bortribromid zugetropft und über Nacht weiter gerührt. Das Gemisch wird langsam mit Wasser versetzt, die organische Phase dreimal mit Wasser gewaschen, über Na₂SO₄ getrocknet, einrotiert und chromatographisch gereinigt. Ausbeute: 104 g (397 mmol), 98 % der Theorie.

Analog können folgende Verbindungen erhalten werden:

| | **Edukt 1** | **Produkt** | **Ausbeute** |
|---|---|---|---|
| e1 | 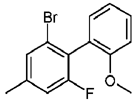 | 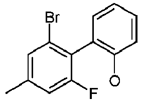 | 92% |
| e2 | 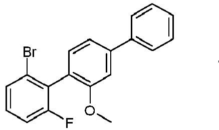 | 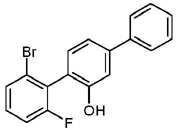 | 90% |
| e3 | 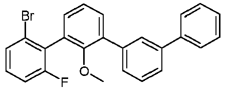 | 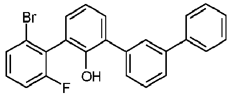 | 93% |
| e4 | 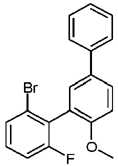 | 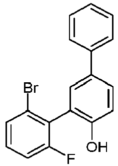 | 94% |

### f) 1-Brom-dibenzofuran

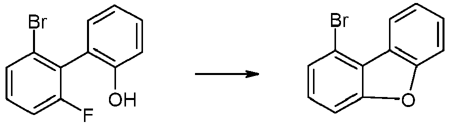

111 g (416 mmol) 6'-Brom-2'-fluor-biphenyl-2-ol werden in 2 L DMF (max. 0.003% H₂O) SeccoSolv^{®} gelöst und auf 5 °C gekühlt. Zu dieser Lösung werden portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, komplett einrotiert und dann chromatographisch gereinigt. Ausbeute: 90 g (367 mmol), 88.5 % der Theorie.

Analog können folgende Verbindungen erhalten werden:

| | **Edukt 1** | **Produkt** | **Ausbeute** |
|---|---|---|---|
| f1 | 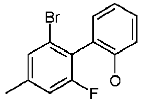 | 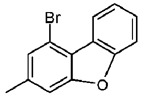 | 81% |
| f2 | 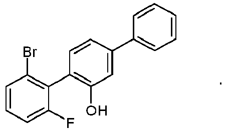 | 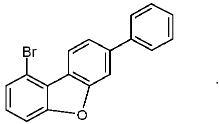 | 78% |
| f3 | 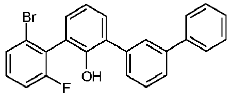 | 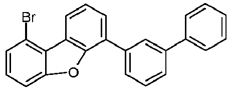 | 73% |
| f4 | 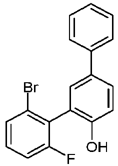 | 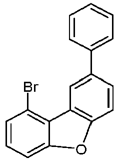 | 79% |

### g) Dibenzofuran-1-boronsäure

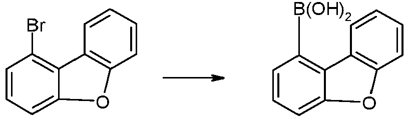

180 g (728 mmol) 1-Brom-dibenzofuran werden in 1500 mL trockenem THF gelöst und auf -78 °C gekühlt. Bei dieser Temperatur wird mit 305 mL (764 mmol / 2.5 M in Hexan) n-Butyllithium innerhalb von ca. 5 min. versetzt und anschließend für 2.5 h bei -78 °C nachgerührt. Bei dieser Temperatur wird mit 151 g (1456 mmol) Borsäuretrimethylester möglichst zügig versetzt und die Reaktion langsam auf RT kommen gelassen (ca. 18 h). Die Reaktionslösung wird mit Wasser gewaschen und der ausgefallene Feststoff und die organische Phase mit Toluol azeotrop getrocknet. Das Rohprodukt wird aus Toluol/Methylenchlorid bei ca. 40 °C ausgerührt und abgesaugt. Ausbeute: 146 g (690 mmol), 95 % der Theorie.

Analog können folgende Verbindungen erhalten werden:

| | **Edukt 1** | **Produkt** | **Ausbeute** |
|---|---|---|---|
| g1 | 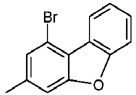 | 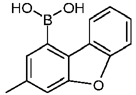 | 81% |
| g2 | 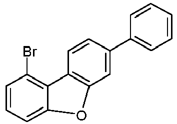 | 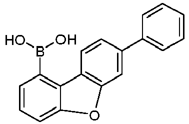 | 78% |
| g3 | 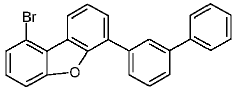 | 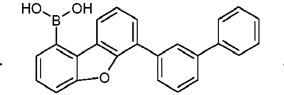 | 73% |
| g4 | 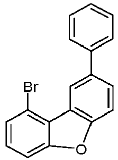 | 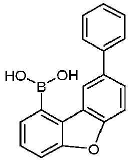 | 79% |
| g5 | 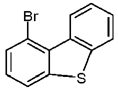 [65642-94-6] | 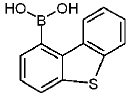 | 73% |

### h) 2-Dibenzofuran-1-yl-4,6-diphenyl-[1,3,5]triazin

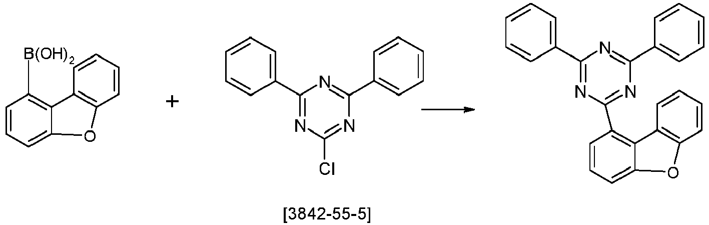

23 g (110.0 mmol) Dibenzofuran-1-boronsäure, 29.5 g (110.0 mmol) 2-Chlor-4,6-diphenyl-1,3,5-triazin und 21 g (210.0 mmol) Natriumcarbonat werden in 500 mL Ethylenglycoldiaminether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan/Heptan umkristallisiert. Die Ausbeute beträgt 37 g (94 mmol), 87 % der Theorie.

Analog können folgende Verbindungen erhalten werden:

| | **Edukt 1** | **Edukt 2** | **Produkt** | **Ausbeute** |
|---|---|---|---|---|
| h1 | 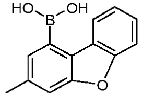 | 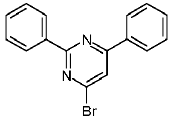 [40734-24-5] | 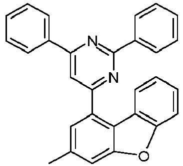 | 73% |
| h2 | 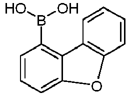 | 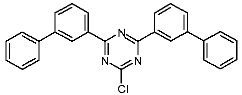 | 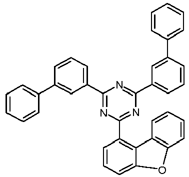 | 82% |
| h3 | 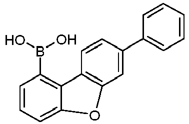 | 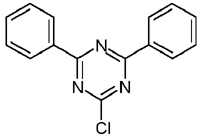 [3842-55-5] | 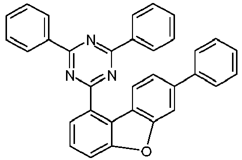 | 73% |
| h4 | 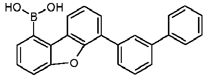 | 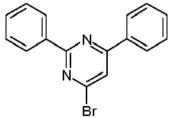 40734-4-5 | 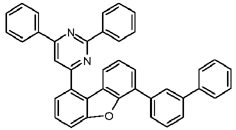 | 72% |
| h5 | 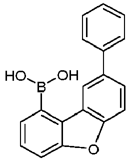 | 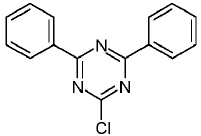 | 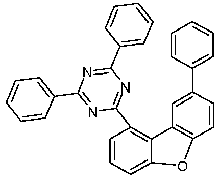 | 65% |
| h6 | 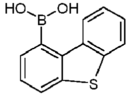 | 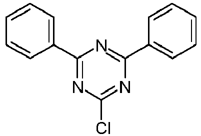 | 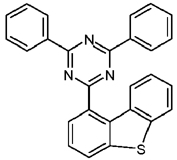 | 63% |
| h14 | 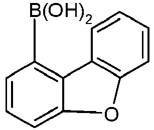 | 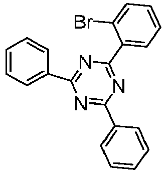 77989-15-2 | 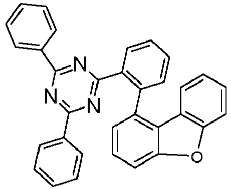 | 77% |
| h15 | 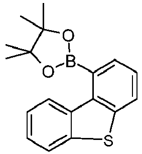 [1434286-69-7] | 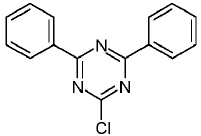 | 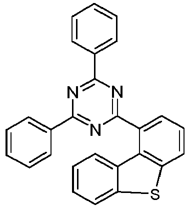 | 76% |
| h16 | 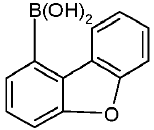 | 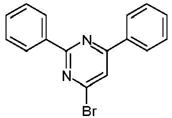 | 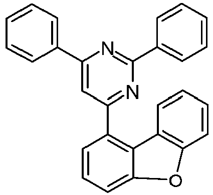 | 68% |

### i) 2-(8-Brom-dibenzofuran-1-yl)-4,6-diphenyl-[1,3,5]triazin

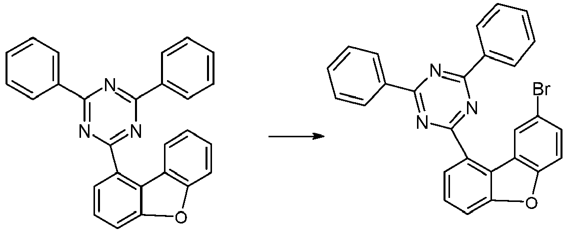

70 g (190.0 mmol) 2-Dibenzofuran-1-yl-4,6-diphenyl-[1,3,5]triazin werden in 2000 mL Essigsäure (100%) und 2000 mL Schwefelsäure (95-98%) suspendiert. Zu dieser Suspension werden portionsweise 34 g (190 mmol) NBS zugegeben und 2 h bei Dunkelheit gerührt. Danach wird mit Wasser/Eis versetzt, der Feststoff abgetrennt und mit Ethanol nachgewaschen. Der Rückstand wird aus Toluol umkristallisiert. Die Ausbeute beträgt 80 g (167 mmol), 87 % der Theorie.

Analog können folgende Verbindungen erhalten werden:

| | **Edukt 1** | **Produkt** | **Ausbeute** |
|---|---|---|---|
| i1 | 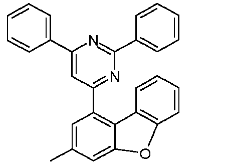 | 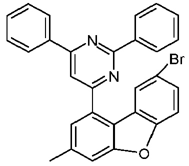 | 80% |
| i2 | 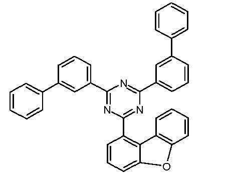 | 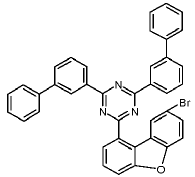 | 41% |
| i3 | 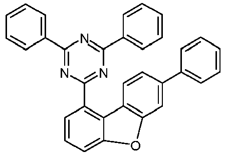 | 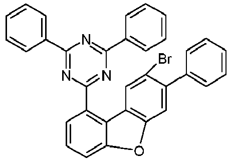 | 52% |
| i4 | 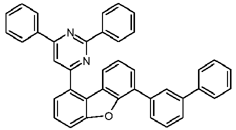 | 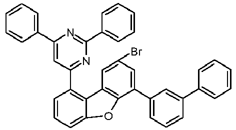 | 64% |
| i5 | 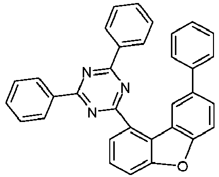 | 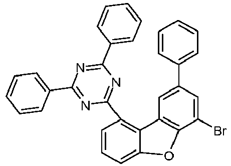 | 33% |
| i6 | 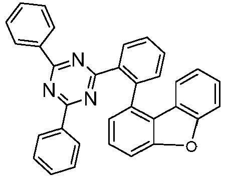 | 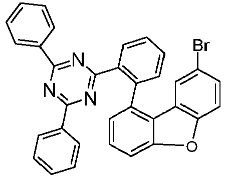 | 41% |
| i7 | 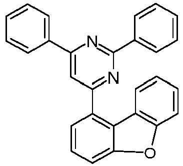 | 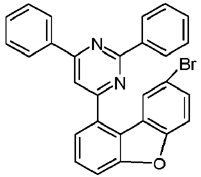 | 58% |

Im Falle von Thiophen-Derivaten wird Nitrobenzol statt Schwefelsäure und elementares Brom an Stelle von NBS eingesetzt:

| | | | |
|---|---|---|---|
| i8 | 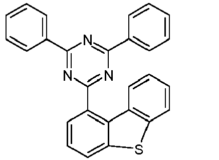 | 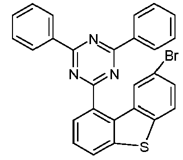 | 55% |

### k) 2,4-Diphenyl-6-[8-(4,4,5,5-tetramethyl-[1,3,2]dioxaborolan-2-yl)-dibenzofuran-1-yl]-[1,3,5]triazin

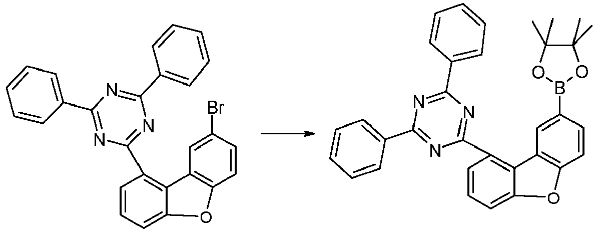

In einem 500 ml-Kolben werden unter Schutzgas 13.3 g (28 mmol) 2-(8-Brom-dibenzofuran-1-yl)-4,6-diphenyl-[1,3,5]triazin und 8.5 g (34 mmol, 1.2 eq.) Bis(pinacolato)-diboran (CAS 73183-34-3) in 120 ml trockenem DMF gelöst und für 30 Minuten entgast. Anschließend werden 8.2 g (84 mmol, 3.0 eq.) Kaliumacetat und 690 mg (0.84 mmol, 3 mol-%) [1,1'-Bis(diphenylphosphino)ferrocen]-dichlorpalladium(II)-Komplex mit Dichlormethan (CAS 95464-05-4) zugegeben und der Ansatz über Nacht auf 90 °C erhitzt. Nach beendeter Reaktion wird mit 300 ml Toluol verdünnt und das Gemisch mit Wasser extrahiert. Der Rückstand wird aus Heptan umkristallisiert. Die Ausbeute beträgt 15 g (24 mmol), 88 % der Theorie.

Analog können folgende Verbindungen erhalten werden:

| | **Edukt 1** | **Produkt** | **Ausbeute** |
|---|---|---|---|
| k1 | 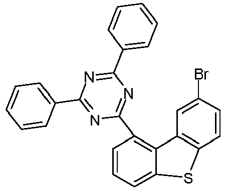 | 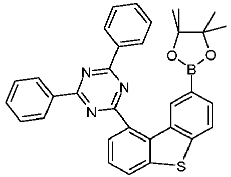 | 80% |
| k2 | 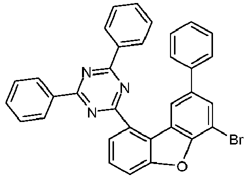 | 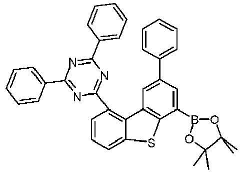 | 64% |
| k3 | 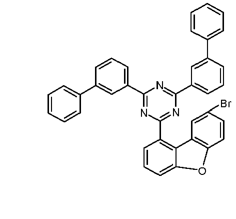 | 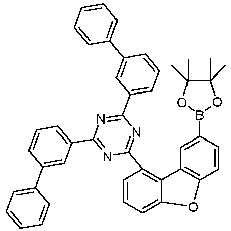 | 63% |
| k4 | 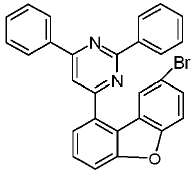 | 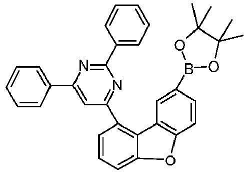 | 67% |
| k5 | 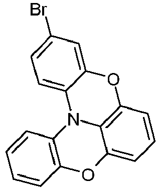 [1416903-68-8] | 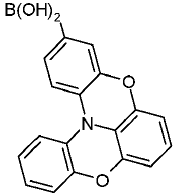 | 65% |
| k6 | 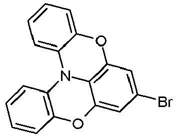 [1333316-19-0] | 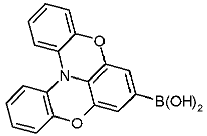 | 67% |
| k7 | 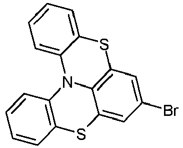 [1333316-19-0] | 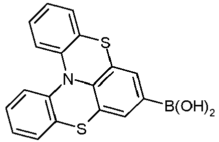 | 60% |

### I) 2-[8-(4-Chlor-phenyl)-dibenzothiophen-1-yl]-4,6-diphenyl-[1,3,5]triazin

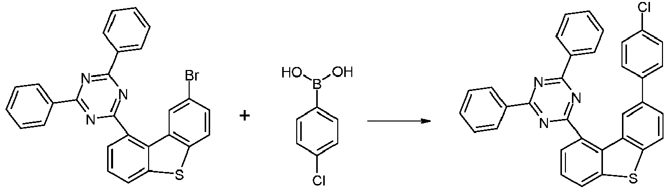

17.6 g (110.0 mmol) 4-Chlorphenyl-1-boronsäure, 54.3 g (110.0 mmol) 2-(8-Brom-dibenzothiophen-1-yl)-4,6-diphenyl-[1,3,5]triazin und 21 g (210.0 mmol) Natriumcarbonat werden in 500 mL Ethylenglycoldimethylether und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan/Heptan umkristallisiert. Die Ausbeute beträgt 46 g (87 mmol), 81 % der Theorie.

Analog können folgende Verbindungen erhalten werden:

| | **Edukt 1** | **Edukt 2** | **Produkt** | **Ausbeute** |
|---|---|---|---|---|
| 1I | 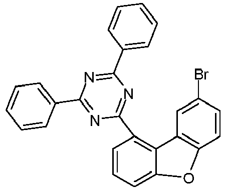 | 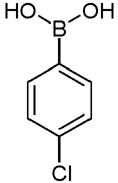 1679-18-1 | 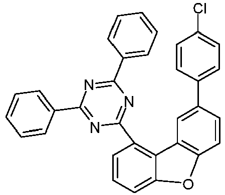 | 73% |
| 2I | 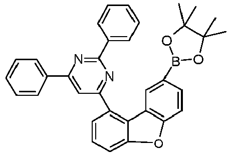 | 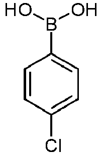 1679-18-1 | 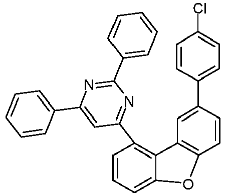 | 82% |

### m) Bihenyl-4-yl-9,9-dimethyl-9H-fluoren-2-yl-amin

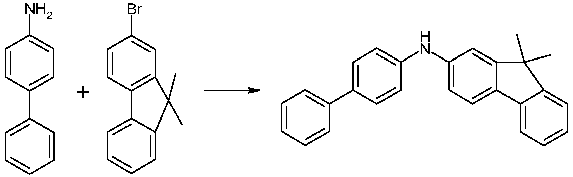

24.0 g (142 mmol, 1.2 eq.) 4-Aminobiphenyl (CAS 92-67-1) und 32.0 g (117 mmol, 1.0 eq.) 2-Brom-9,9'-dimethylfluoren (CAS 28320-31-2) werden in 950 ml Toluol vorgelegt und 30 Minuten mit Argon gesättigt. Anschließend werden 1.0 g (1.8 mmol, 0.02 eq.) 1,1'-Bis(diphenyl-phosphino)ferrocen (CAS 12150-46-8), 350 mg (1.6 mmol, 0.01 eq.) Palladium(II)-acetat (CAS 3375-31-3) und 29 g (300 mmol, 2.6 eq.) Natrium-*tert*-butylat (CAS 865-48-5) zugegeben und über Nacht unter Rückfluss erhitzt. Nach beendeter Reaktion wird der Ansatz mit 300 ml Toluol verdünnt und mit Wasser extrahiert. Die organische Phase wird über Natriumsulfat getrocknet und das Lösungsmittel am Rotationsverdampfer entfernt. Das braune Öl wird mit 50 ml Ethylacetat versetzt und in eine Mischung aus Heptan/Essigester 20:1 gegeben. Der entstehende Feststoff wird abgesaugt und mit Heptan gewaschen. Die Ausbeute beträgt 29 g (80 mmol), 69 % der Theorie.

Analog können folgende Verbindungen erhalten werden:

| | **Edukt 1** | **Edukt 2** | **Produkt** | **Ausbeute** |
|---|---|---|---|---|
| 1m | 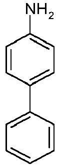 92-67-1 | 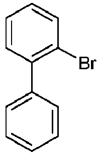 2052-07-5 | 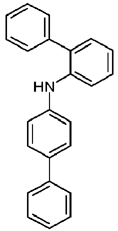 | 71% |
| 2m | 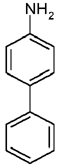 92-67-1 | 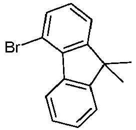 942615-32-9 | 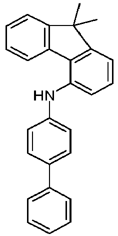 | 61% |
| 3m | 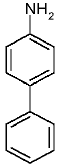 92-67-1 | 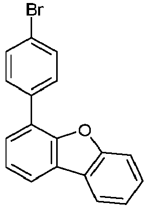 955959-84-9 | 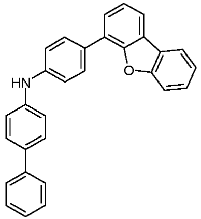 | 78% |
| 4m | 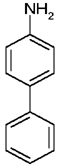 92-67-1 | 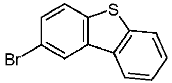 22439-61-8 | 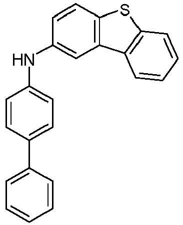 | 82% |
| 5m | 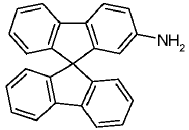 118951-68-1 | 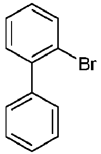 2052-07-5 | 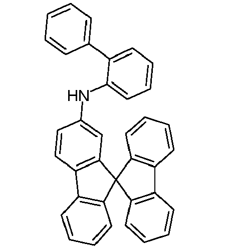 | 62% |
| 6m | 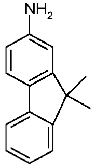 108714-73-4 | 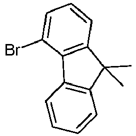 942615-32-9 | 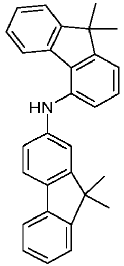 | 47% |
| 7m | 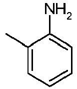 95-53-4 | 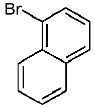 90-11-9 | 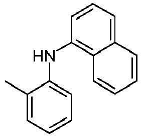 | 92% |
| 8m | 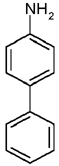 92-67-1 | 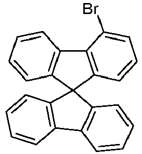 171408-76-7 | 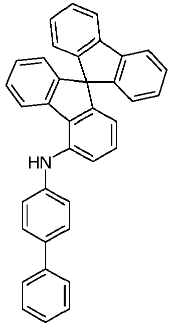 | 75% |
| 9m | 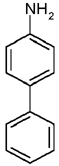 92-67-1 | 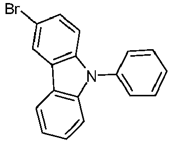 1153-85-1 | 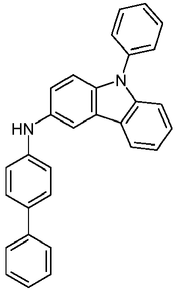 | 84% |
| 10m | 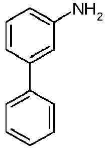 90-41-5 | 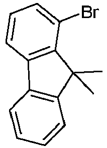 1225053-54-2 | 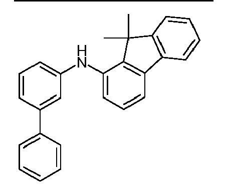 | 62% |
| 11m | 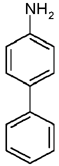 92-67-1 | 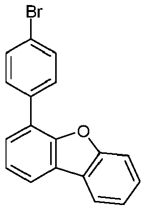 Br 1028647-93-9 | 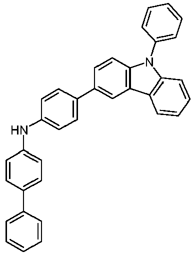 | 78% |
| 12m | 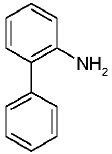 90-41-5 | 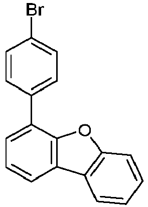 955959-84-9 | 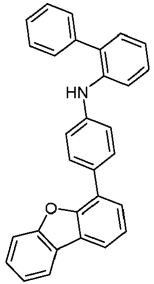 | 62% |

### n) Biphenyl-4-yl-(9,9-dimethyl-9H-fluoren-2-yl)-{4-[9-(4,6-diphenyl-[1,3,5]triazin-2-yl)-dibenzofuran-2-yl]-phenyl}-amin

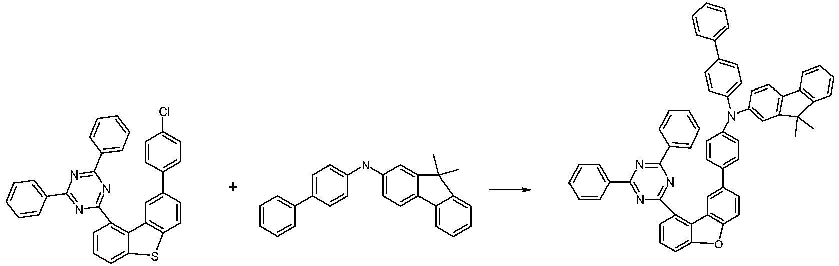

27.8 g (80 mmol, 1.0 eq.) Biphenyl-4-yl-(9,9-dimethyl-9H-fluoren-2-yl)-amin und 42 g (80 mmol, 1.0 eq.) 2-[8-(4-Chlorphenyl)-dibenzofuran-1-yl]-4,6-diphenyl-[1,3,5]triazin werden in 600 ml Toluol gelöst und für 30 Minuten entgast. Anschließend werden 45 g (240 mmol, 3.0 eq.) Natriumtert-butylat, 890 mg (0.40 mmol, 0.050 eq.) Palladium(II)-acetat und 8 ml (8.0 mmol, 0.10 eq.) einer 1M Tri-*tert*-butylphosphin-Lösung zugegeben. Der Ansatz wird über Nacht unter Rückfluss erhitzt und nach beendeter Reaktion zweimal über Aluminiumoxid mit Toluol filtriert. Nach Entfernen des Lösungsmittels am Rotationsverdampfer wird das Öl in etwas THF gelöst und in Heptan eingetragen. Der Rückstand wird aus Toluol und aus Heptan/Toluol 1:1 umkristallisiert und abschließend im Hochvakuum (p = 5 × 10⁻⁵ mbar, T = 350 °C) sublimiert. Die Ausbeute beträgt 50 g (59 mmol), 75 % der Theorie.

Analog können folgende Verbindungen erhalten werden:

| | **Edukt 3** | **Edukt 4** | **Produkt** | **Ausbeute** |
|---|---|---|---|---|
| 1n | 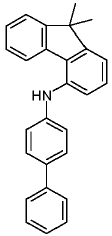 | 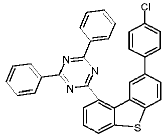 | 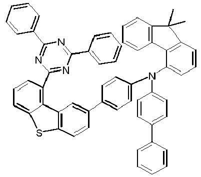 | 69% |
| 2n | 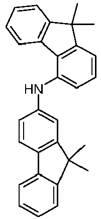 | 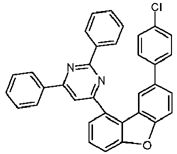 | 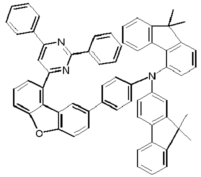 | 64% |
| 3n | 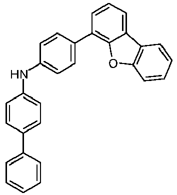 | 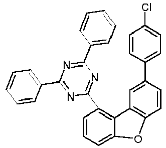 | 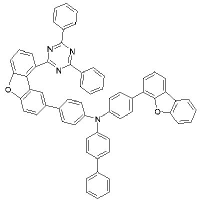 | 59% |
| 4n | 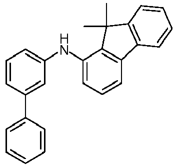 | 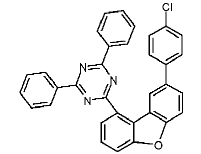 | 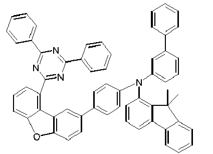 | 61% |
| 5n | 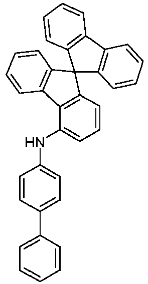 | 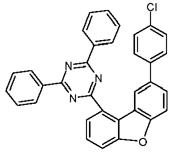 | 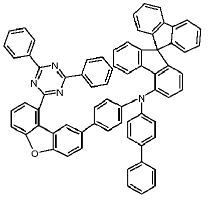 | 67% |
| 6n | 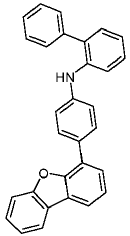 | 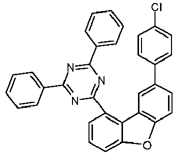 | 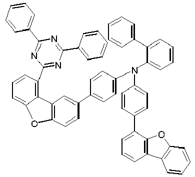 | 60% |
| 7n | 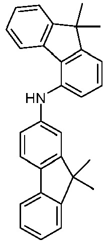 | 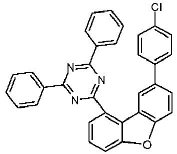 | 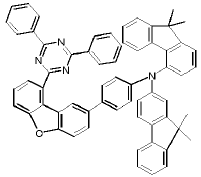 | 72% |

### Analog können folgende Verbindungen erhalten werden:

| | **Edukt 3** | **Edukt 4** | **Produkt** | **Ausbeute** |
|---|---|---|---|---|
| 8n | 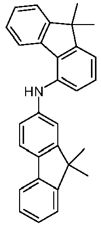 | 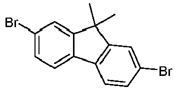 28320-32-3 | 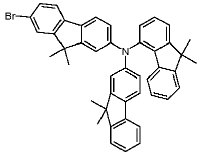 | 51% |
| 9n | 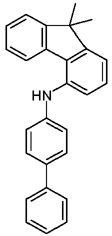 | 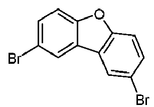 10016-52-1 | 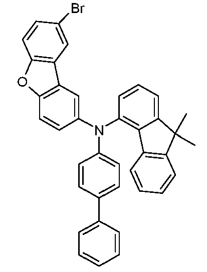 | 37% |
| 10n | 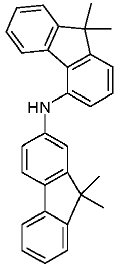 | 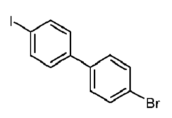 105946-82-5 | 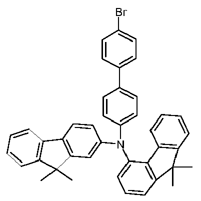 | 80% |
| 11n | 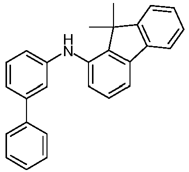 | 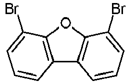 201138-91-2 | 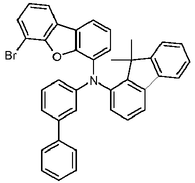 | 36% |
| 12n | 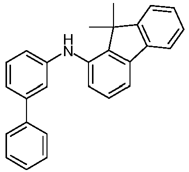 | 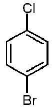 | 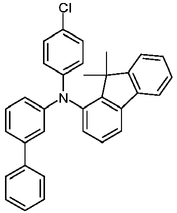 | 48% |
| 13n | 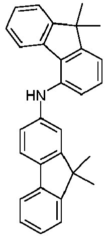 | 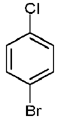 | 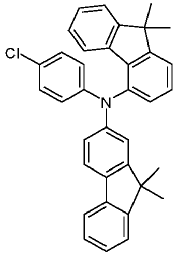 | 50% |
| 14n | 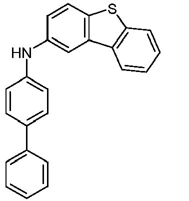 | 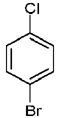 | 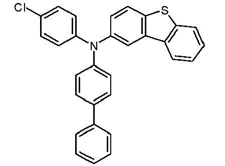 | 47% |

### o) (9,9-Dimethyl-9H-fluoren-4-yl)-(9,9-dimethyl-9H-fluoren-2-yl)-{7-[9-(4,6-diphenyl-[1,3,5]triazin-2-yl)-dibenzofuran-2-yl]-9,9-dimethyl-9H-fluoren-2-yl}-amin

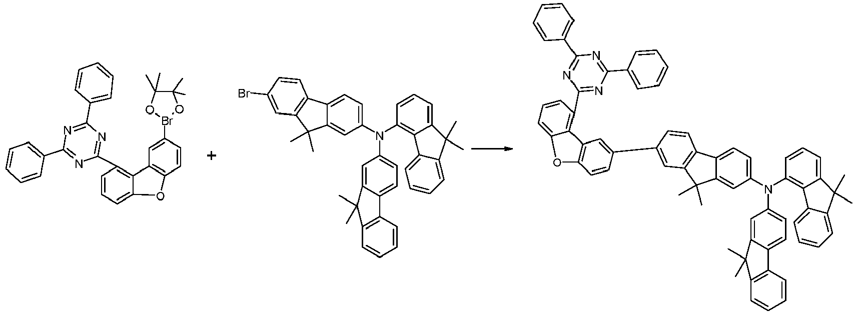

84 g (125 mmol, 1.0 eq.) (7-Brom-9,9-dimethyl-9H-fluoren-2-yl)-(9,9-dimethyl-9H-fluoren-4-yl)-(9,9-dimethyl-9H-fluoren-2-yl)-amin und 72 g (125 mmol, 1.0 eq) 2,4-Diphenyl-6-[8-(4,4,5,5-tetramethyl-[1,3,2]dioxa-borolan-2-yl)-dibenzofuran-1-yl]-[1,3,5]triazin werden in einem Gemisch aus 400 ml Wasser, 400 ml Dioxan und 400 ml Toluol vorgelegt und für 30 Minuten entgast. Nach Zugabe von 280 mg (1.25 mmol, 1 mol-%) Palladium(II)-acetat und 1.14 g (3.75 mmol, 3 mol-%) Tri-o-tolylphoshin wird der Ansatz über Nacht unter Rückfluss erhitzt und nach beendeter Reaktion mit etwas Wasser versetzt. Die organische Phase wird abgetrennt und zweimal mit Wasser extrahiert. Nach Trocknen der organischen Phase über Natriumsulfat wird der Rückstand aus Heptan/Toluol umkristallisiert. Der Rückstand wird aus Toluol und aus Heptan/Toluol 1:1 umkristallisiert und abschließend im Hochvakuum (p = 5 × 10⁻⁵ mbar, T = 350 °C) sublimiert. Es werden 96 g (96 mmol, 80%) eines beigen Feststoffs erhalten.

Analog können folgende Verbindungen erhalten werden:

| | **Edukt 3** | **Edukt 4** | **Produkt** | **Ausbeute** |
|---|---|---|---|---|
| o1 | 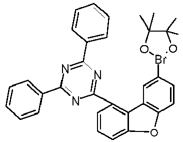 | 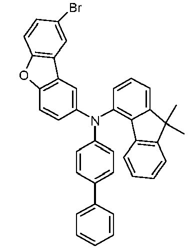 | 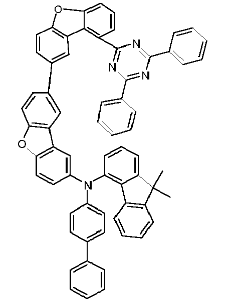 | 51% |
| o2 | 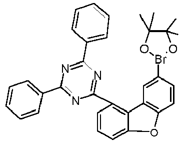 | 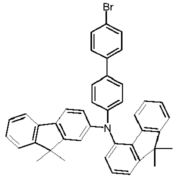 | 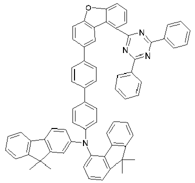 | 57% |
| o3 | 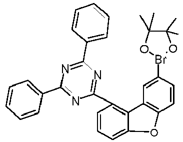 | 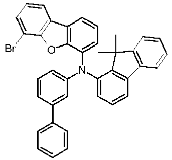 | 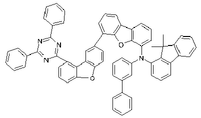 | 56% |
| o4 | 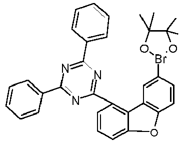 | 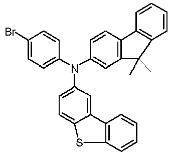 | 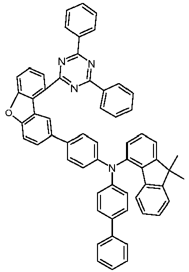 | 61% |
| o5 | 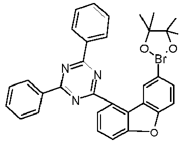 | 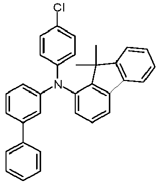 | 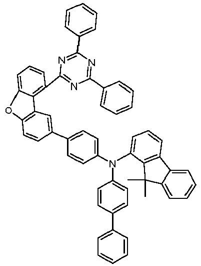 | 63% |
| o6 | 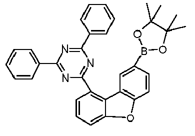 | 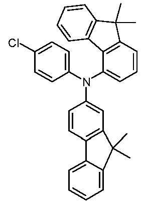 | 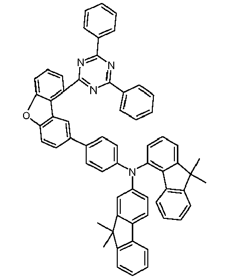 | 57% |
| o7 | 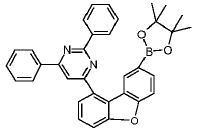 | 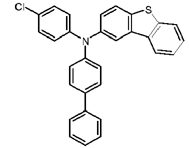 | 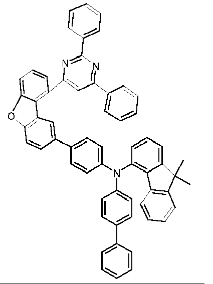 | 62% |
| o8 | 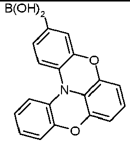 | 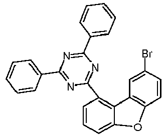 | 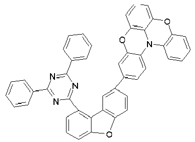 | 63% |
| o9 | 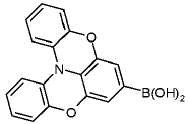 | 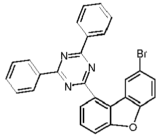 | 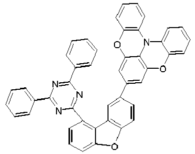 | 64% |
| o10 | 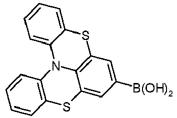 | 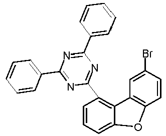 | 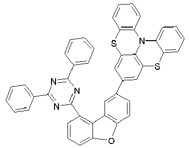 | 60% |

### Herstellung der OLEDs

In den folgenden Beispielen E1 bis E12 (siehe Tabelle 1) wird der Einsatz der erfindungsgemäßen Materialien in OLEDs vorgestellt.

**Vorbehandlung für die Beispiele E1-E12:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie EG3:IC1:TEG1 (59%:29%:12%) bedeutet hierbei, dass das Material EG3 in einem Volumenanteil von 59%, IC1 in einem Anteil von 29% und TEG1 in einem Anteil von 12% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Spannung, die Stromeffizienz sowie die externe Quanteneffizienz werden für eine Leuchtdichte von 1000 cd/m² angegeben. Die Elektrolumineszenzspektren werden bei einer Leuchtdichte von 1000 cd/m² bestimmt und daraus die CIE 1931 × und y Farbkoordinaten berechnet.

Als Lebensdauer LD_L1 wird die Zeit definiert, nach der die Leuchtdichte bei Betrieb mit konstantem Strom von der Startleuchtdichte auf einen gewissen Anteil L1 absinkt. LD_80 stellt also die Zeit dar, in der die Leuchtdichte bei Betrieb auf 80% ihres Anfangswertes absinkt. In den unten aufgeführten Beispielen wird als Betriebsbedingung eine Stromdichte von 40 mA/cm² verwendet.

### Verwendung von erfindungsgemäßen Materialien in OLEDs

Die erfindungsgemäßen Verbindungen EG1 bis EG12 werden in den Beispielen E1 bis E12 als Matrixmaterial in der Emissionsschicht eingesetzt. Die Farbkoordinaten der Elektrolumineszenzspektren der OLEDs aus diesen Versuchen liegen bei CIEx=0.3 und CIEy= 0.6. Somit eignen sich die Materialien für den Einsatz in der Emissionsschicht von phosphoreszierenden grünen OLEDs.

Mit der erfindungsgemäßen OLED in Beispiel E1 werden dabei die folgenden Devicedaten erhalten:
Spannung: 3.2 V
Stromeffizienz: 62 cd/A
EQE: 17%
LD_80: 160 h

Mit den erfindungsgemäßen OLEDs in den Beispielen E2 bis E12 werden vergleichbare Devicedaten erhalten.

**Tabelle 1: Aufbau der OLEDs**

| Bsp | HIL Dicke | HTL Dicke | EBL Dicke | EML Dicke | HBL | ETL Dicke |
|---|---|---|---|---|---|---|
| E1 | HATCN 5nm | SpMA1 230nm | SpMA3 20nm | EG3:IC1:TEG1 (59%:29%:12%) 30nm | --- | ETM1:LiQ (50%:50%) 40nm |
| E2 | HATCN 5nm | SpMA1 230nm | SpMA3 20nm | EG1:TEG1 (95%:5%) 30nm | --- | ETM1:LiQ (50%:50%) 40nm |
| E3 | HATCN 5nm | SpMA1 230nm | SpMA3 20nm | EG2:TEG1 (95%:5%) 30nm | --- | ETM1:LiQ (50%:50%) 40nm |
| E4 | HATCN 5nm | SpMA1 230nm | SpMA3 20nm | EG3:TEG1 (95%:5%) 30nm | --- | ETM1:LiQ (50%:50%) 40nm |
| E5 | HATCN 5nm | SpMA1 230nm | SpMA3 20nm | EG4:TEG1 (95%:5%) 30nm | --- | ETM1:LiQ (50%:50%) 40nm |
| E6 | HATCN 5nm | SpMA1 230nm | SpMA3 20nm | EG5:TEG1 (95%:5%) 30nm | --- | ETM1:LiQ (50%:50%) 40nm |
| E7 | HATCN 5nm | SpMA1 230nm | SpMA3 20nm | EG6:TEG1 (95%:5%) 30nm | --- | ETM1:LiQ (50%:50%) 40nm |
| E8 | HATCN 5nm | SpMA1 230nm | SpMA3 20nm | EG7:TEG1 (95%:5%) 30nm | --- | ETM1:LiQ (50%:50%) 40nm |
| E9 | HATCN 5nm | SpMA1 230nm | SpMA3 20nm | EG8:TEG1 (95%:5%) 30nm | --- | ETM1:LiQ (50%:50%) 40nm |
| E10 | HATCN 5nm | SpMA1 230nm | SpMA3 20nm | EG9:TEG1 (95%:5%) 30nm | --- | ETM1:LiQ (50%:50%) 40nm |
| E11 | HATCN 5nm | SpMA1 230nm | SpMA3 20nm | EG10:TEG1 (95%:5%) 30nm | --- | ETM1:LiQ (50%:50%) 40nm |
| E12 | HATCN 5nm | SpMA1 230nm | SpMA3 20nm | EG11:TEG1 (95%:5%) 30nm | --- | ETM1:LiQ (50%:50%) 40nm |

**Tabelle 2: Strukturformeln der Materialien für die OLEDs**

| | |
|---|---|
| 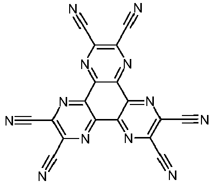 | 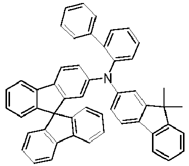 |
| HATCN | SpMA1 |
| 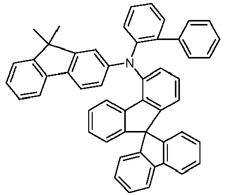 | 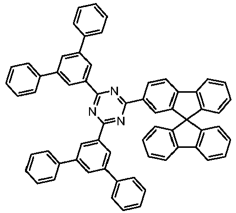 |
| SpMA3 | ETM1 |
| 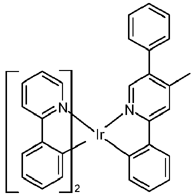 | 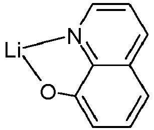 |
| TEG1 | LiQ |
| 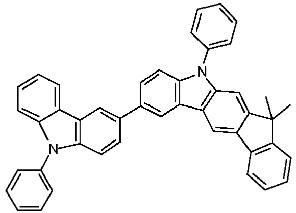 | |
| IC1 | |
| 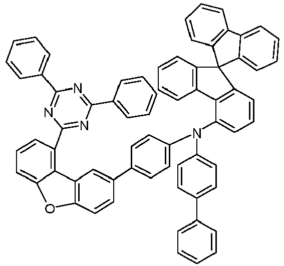 | 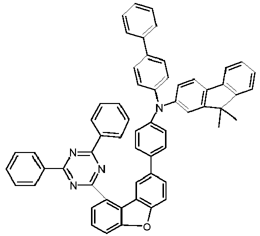 |
| EG1 | EG2 |
| 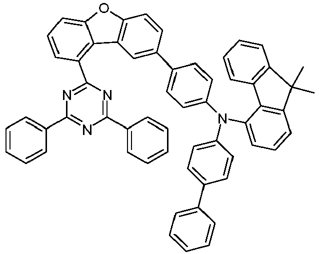 | 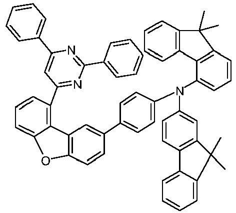 |
| EG3 | EG4 |
| 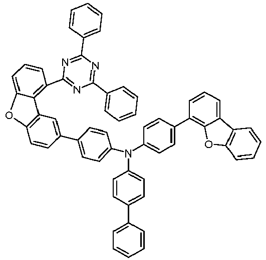 | 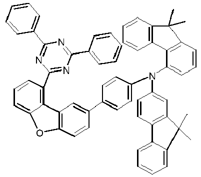 |
| EG5 | EG6 |
| 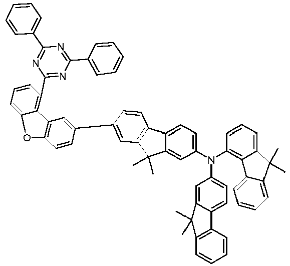 | 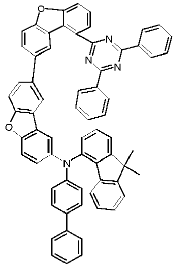 |
| EG7 | EG8 |
| 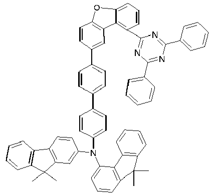 | 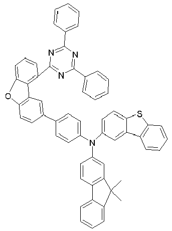 |
| EG9 | EG10 |
| 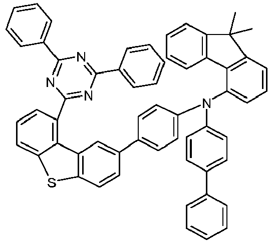 | |
| EG11 | |

## Patentansprüche

1. Verbindung gemäß Formel (1), 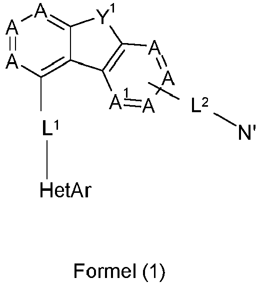 wobei für die verwendeten Symbole gilt:
A, A¹ ist bei jedem Auftreten gleich oder verschieden CR¹ oder N, wobei von allen A und A¹ maximal zwei Gruppen A und/oder A¹ pro Cyclus für N stehen und jenes A für C steht, an welches L² gebunden ist;
Y¹ ist O, S oder NR, wobei der Rest R, der an N gebunden ist, ungleich H oder D ist;
L¹ ist eine Einfachbindung oder ein aromatisches oder heteroaromatisches Ringsystem mit 5 bis 30 aromatischen Ringatomen, das durch einen oder mehrere Reste R³ substituiert sein kann;
L² ist eine Einfachbindung oder ein aromatisches oder heteroaromatisches Ringsystem mit 5 bis 30 aromatischen Ringatomen, das durch einen oder mehrere Reste R³ substituiert sein kann;
N' ist eine Gruppe der Formel (L-1), 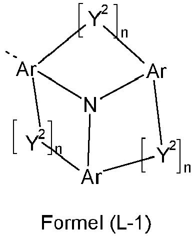
wobei die gestrichelte Bindung die Verknüpfung mit L² darstellt;
Y² ist bei jedem Auftreten gleich oder verschieden gewählt aus einer Einfachbindung, C(R)₂, C=O, O, S, S=O, SO₂, Si(R)₂, NR, wobei der Rest R, der an N gebunden ist, ungleich H oder D ist;
n ist bei jedem Auftreten gleich oder verschieden 0 oder 1, wobei wenn mindestens ein Y² für eine Einfachbindung steht, mindestens zwei n gleich 1 sind;
Ar ist bei jedem Auftreten gleich oder verschieden ein aromatisches oder heteroaromatisches Ringsystem mit 5 bis 30 aromatischen Ringatomen, welches durch einen oder mehrere Reste R³ substituiert sein kann;
HetAr ist eine Gruppe der Formel (2), (3) oder (4), 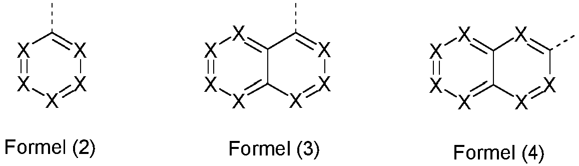 wobei die gestrichelte Bindung die Verknüpfung dieser Gruppe darstellt;
X ist bei jedem Auftreten gleich oder verschieden CR² oder N mit der Maßgabe, dass mindestens ein Symbol X für N steht;
R, R¹, R², R³ ist bei jedem Auftreten gleich oder verschieden ausgewählt aus der Gruppe bestehend aus H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, Si(Ar¹)₃, Si(R⁴)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁴ substituiert sein kann, wobei eine oder mehrere nicht-benachbarte CH₂-Gruppen durch R⁴C=CR⁴, Si(R⁴)₂, C=O, C=NR⁴, P(=O)(R⁴), SO, SO₂, NR⁴, O, S oder CONR⁴ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁴ substituiert sein kann, einer Aryloxy- oder Heteroaryloxygruppe mit 5 bis 40 aromatischen Ringatomen, die mit einem oder mehreren Resten R⁴ substituiert sein kann, oder einer Aralkyl- oder Heteroaralkylgruppe mit 5 bis 40 aromatischen Ringatomen, die mit einem oder mehreren Resten R⁴ substituiert sein kann; dabei können optional zwei benachbarte Substituenten R, bzw. zwei benachbarte Substituenten R¹, bzw. zwei benachbarte Substituenten R², bzw. zwei benachbarte Substituenten R³ ein aliphatisches, heteroaliphatisches, aromatisches oder heteroaromatisches Ringsystem bilden, das mit einem oder mehreren Resten R⁴ substituiert sein kann;
Ar¹ ist bei jedem Auftreten gleich oder verschieden ein aromatisches oder heteroaromatisches Ringsystem mit 5 bis 30 aromatischen Ringatomen, das mit einem oder mehreren nicht-aromatischen Resten R⁴ substituiert sein kann; dabei können zwei Reste Ar¹, welche an dasselbe N-Atom, P-Atom oder B-Atom binden, auch durch eine Einfachbindung oder eine Brücke, ausgewählt aus N(R⁴), C(R⁴)₂, O oder S, miteinander verbrückt sein;
R⁴ ist bei jedem Auftreten gleich oder verschieden ausgewählt aus der Gruppe bestehend aus H, D, F, Cl, Br, I, CN, NO₂,N(R⁵)₂, C(=O)R⁵, Si(R⁵)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R⁵ substituiert sein kann, wobei eine oder mehrere nicht-benachbarte CH₂-Gruppen durch R⁵C=CR⁵, Si(R⁵)₂, C=O, C=NR⁵, P(=O)(R⁵), SO, SO₂, NR⁵, O, S oder CONR⁵ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R⁵ substituiert sein kann, einer Aryloxy- oder Heteroaryloxygruppe mit 5 bis 40 aromatischen Ringatomen, die mit einem oder mehreren Resten R⁵ substituiert sein kann, oder einer Aralkyl- oder Heteroaralkylgruppe mit 5 bis 40 aromatischen Ringatomen, die mit einem oder mehreren Resten R⁵ substituiert sein kann; dabei können optional zwei benachbarte Substituenten R⁴ ein aliphatisches oder aromatisches oder heteroaromatisches Ringsystem bilden, das mit einem oder mehreren Resten R⁵ substituiert sein kann;
R⁵ ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, Cl, Br, I oder CN ersetzt sein können und das durch ein oder mehrere Alkylgruppen mit jeweils 1 bis 4 Kohlenstoffatomen substituiert sein kann; dabei können zwei oder mehr benachbarte Substituenten R⁵ miteinander ein aliphatisches Ringsystem bilden;
wobei folgende Verbindungen ausgenommen sind:

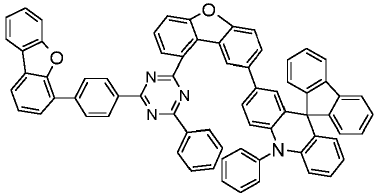

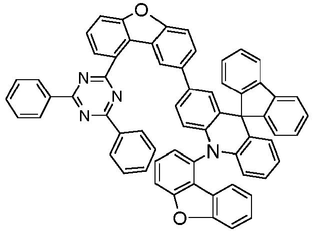

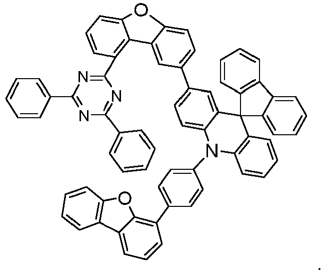

2. Verbindung nach Anspruch 1 gemäß Formel (1a), 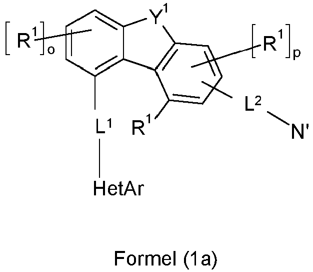 wobei die verwendeten Symbole die oben genannten Bedeutungen aufweisen und o für 0, 1, 2 oder 3 steht und p für 0, 1, oder 2 steht.

3. Verbindung nach Anspruch 1 oder 2 gemäß einer der Formeln (1b) bis (1d), 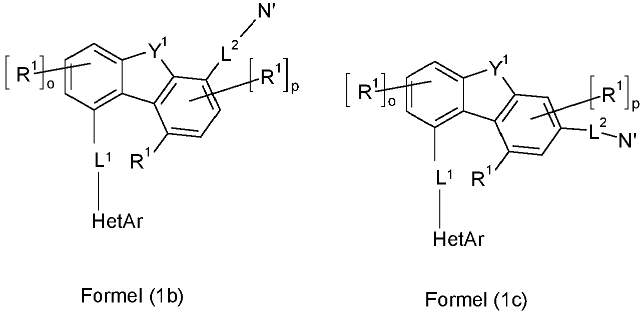 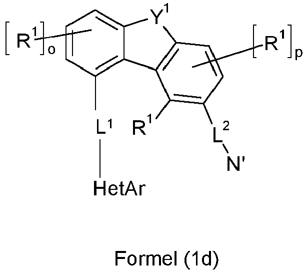 wobei die verwendeten Symbole die in Anspruch 1 und 2 genannten Bedeutungen aufweisen.

4. Verbindung nach einem oder mehreren der Ansprüche 1 bis 3, **dadurch gekennzeichnet, dass** die Gruppen der Formeln (2), (3) und (4) ausgewählt sind aus den Gruppen der Formeln (2-1) bis (2-10), (3-1) und (4-1), 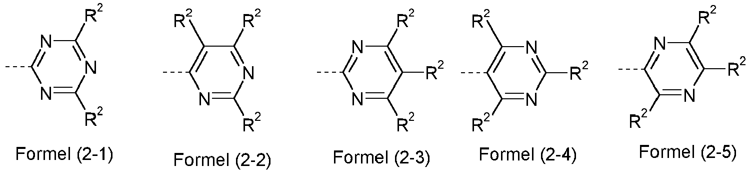 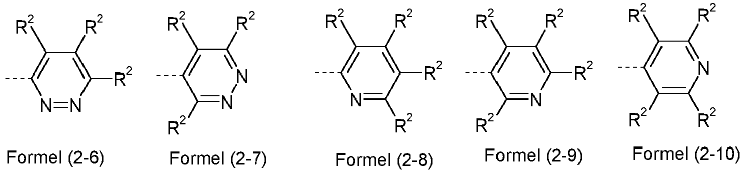 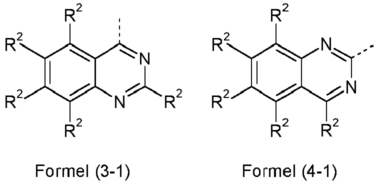 wobei die gestrichelte Bindung die Verknüpfung dieser Gruppen darstellt und R² die in Anspruch 1 genannten Bedeutungen aufweist.

5. Verbindung nach einem oder mehreren der Ansprüche 1 bis 4, **dadurch gekennzeichnet, dass** Ar gleich oder verschieden bei jedem Auftreten für ein aromatisches oder heteroaromatisches Ringsystem mit 6 bis 18 aromatischen Ringatomen steht, das jeweils durch einen oder mehrere Reste R³ substituiert sein kann.

6. Verbindung nach einem oder mehreren der Ansprüche 1 bis 5, **dadurch gekennzeichnet, dass** die Gruppe N' eine Gruppe der Formeln (L-2) bis (L-7) ist, 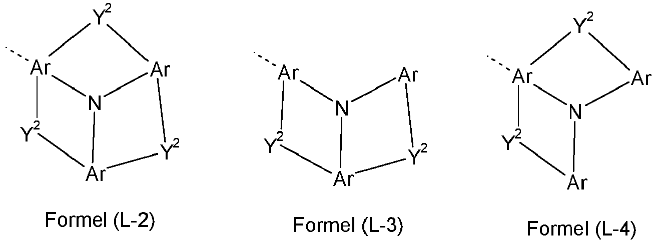 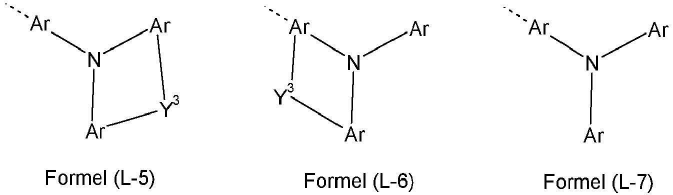 wobei die auftretenden Symbole die in Anspruch 1 genannten Bedeutungen aufweisen und zusätzlich gilt:
Y³ ist bei jedem Auftreten gleich oder verschieden gewählt aus C(R)₂, C=O, O, S, S=O, SO₂, Si(R)₂, NR, wobei der Rest R, der an N gebunden ist, ungleich H oder D ist.

7. Verbindung nach Anspruch 6, **dadurch gekennzeichnet, dass** die Gruppe N' eine Gruppe der Formel (L-7) ist.

8. Verbindung nach Anspruch 6, **dadurch gekennzeichnet, dass** Y³ bei jedem Auftreten gleich oder verschieden gewählt ist aus C=O, O, S, S=O, SO₂, Si(R)₂, NR, wobei der Rest R, der an N gebunden ist, ungleich H oder D ist.

9. Verbindung nach einem oder mehreren der Ansprüche 1 bis 8, **dadurch gekennzeichnet, dass** Y¹ für O oder S steht.

10. Verfahren zur Herstellung einer Verbindung nach einem oder mehreren der Ansprüche 1 bis 9 ausgehend von 1-Halogen-dibenzofuran bzw. 1-Halogen-dibenzothiophen, **gekennzeichnet durch** die folgenden Schritte:
(1) optional Umsetzung der Halogengruppe zu einer Boronsäure bzw. einem Boronsäurederivat;
(2) Einführung der Gruppe HetAr oder L¹-HetAr durch eine Kupplungsreaktion;
(3) Halogenierung, insbesondere Bromierung, des Dibenzofurans bzw. Dibenzothiophens in 8-Position;
(4) Einführung der Gruppe N' durch eine Kupplungsreaktion.

11. Formulierung, enthaltend mindestens eine Verbindung nach einem oder mehreren der Ansprüche 1 bis 9 und mindestens ein Lösemittel.

12. Verwendung einer Verbindung nach einem oder mehreren der Ansprüche 1 bis 9 in einer elektronischen Vorrichtung.

13. Elektronische Vorrichtung enthaltend mindestens eineVerbindung nach einem oder mehreren der Ansprüche 1 bis 9.

14. Elektronische Vorrichtung nach Anspruch 13, wobei es sich um eine organische Elektrolumineszenzvorrichtung handelt, **dadurch gekennzeichnet, dass** die Verbindung nach einem oder mehreren der Ansprüche 1 bis 9 als Matrixmaterial für fluoreszierende oder phosphoreszierende Emitter und/oder in einer Elektronentransportschicht und/oder in einer elektronenblockierenden bzw. exzitonenblockierenden Schicht und/oder in einer Lochtransportschicht eingesetzt wird.

## Claims

1. Compound of formula (1) 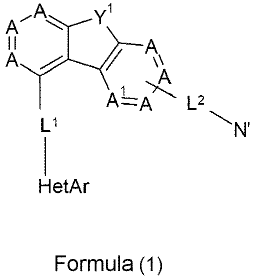 where the symbols used are as follows:
A, A¹ is the same or different at each instance and is CR¹ or N, where, of all A and A¹, not more than two A and/or A¹ groups per cycle are N and that A to which L² is bonded is C;
Y¹ is O, S or NR, where the R radical bonded to N is not H or D;
L¹ is a single bond or an aromatic or heteroaromatic ring system which has 5 to 30 aromatic ring atoms and may be substituted by one or more R³ radicals;
L² is a single bond or an aromatic or heteroaromatic ring system which has 5 to 30 aromatic ring atoms and may be substituted by one or more R³ radicals;
N' is a group of the formula (L-1) 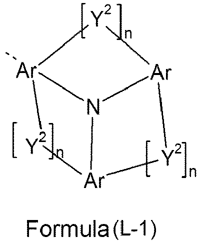
where the dotted bond represents the linkage to L²;
Y² is the same or different at each instance and is selected from a single bond, C(R)₂, C=O, O, S, S=O, SO₂, Si(R)₂, NR, where the R radical bonded to N is not H or D;
n is the same or different at each instance and is 0 or 1, where, when at least one Y² is a single bond, at least two n are 1;
Ar is the same or different at each instance and is an aromatic or heteroaromatic ring system which has 5 to 30 aromatic ring atoms and may be substituted by one or more R³ radicals;
HetAr is a group of the formula (2), (3) or (4) 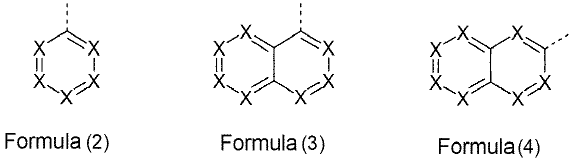 where the dotted bond represents the linkage of this group;
X is the same or different at each instance and is CR² or N, with the proviso that at least one X symbol is N;
R, R¹, R², R³ is the same or different at each instance and is selected from the group consisting of H, D, F, Cl, Br, I, CN, NO₂, N(Ar¹)₂, N(R⁴)₂, C(=O)Ar¹, C(=O)R⁴, P(=O)(Ar¹)₂, P(Ar¹)₂, B(Ar¹)₂, Si(Ar¹)₃, Si(R⁴)₃, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁴ radicals, where one or more nonadjacent CH₂ groups may be replaced by R⁴C=CR⁴, Si(R⁴)₂, C=O, C=NR⁴, P(=O)(R⁴), SO, SO₂, NR⁴, O, S or CONR⁴ and where one or more hydrogen atoms may be replaced by D, F, Cl, Br, I, CN or NO₂,an aromatic or heteroaromatic ring system which has 5 to 40 aromatic ring atoms and may be substituted in each case by one or more R⁴ radicals, an aryloxy or heteroaryloxy group which has 5 to 40 aromatic ring atoms and may be substituted by one or more R⁴ radicals, and an aralkyl or heteroaralkyl group which has 5 to 40 aromatic ring atoms and may be substituted by one or more R⁴ radicals; at the same time, it is optionally possible for two adjacent R substituents, or two adjacent R¹ substituents, or two adjacent R² substituents, or two adjacent R³ substituents to form an aliphatic, heteroaliphatic, aromatic or heteroaromatic ring system which may be substituted by one or more R⁴ radicals;
Ar¹ is the same or different at each instance and is an aromatic or heteroaromatic ring system which has 5 to 30 aromatic ring atoms and may be substituted by one or more nonaromatic R⁴ radicals; at the same time, two Ar¹ radicals bonded to the same nitrogen atom, phosphorus atom or boron atom may also be bridged to one another by a single bond or a bridge selected from N(R⁴), C(R⁴)₂, O and S;
R⁴ is the same or different at each instance and is selected from the group consisting of H, D, F, Cl, Br, I, CN, NO₂,N(R⁵)₂, C(=O)R⁵, Si(R⁵)₃, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R⁵ radicals, where one or more nonadjacent CH₂ groups may be replaced by R⁵C=CR⁵, Si(R⁵)₂, C=O, C=NR⁵, P(=O)(R⁵), SO, SO₂, NR⁵, O, S or CONR⁵ and where one or more hydrogen atoms may be replaced by D, F, Cl, Br, I, CN or NO₂,an aromatic or heteroaromatic ring system which has 5 to 40 aromatic ring atoms and may be substituted in each case by one or more R⁵ radicals, an aryloxy or heteroaryloxy group which has 5 to 40 aromatic ring atoms and may be substituted by one or more R⁵ radicals, and an aralkyl or heteroaralkyl group which has 5 to 40 aromatic ring atoms and may be substituted by one or more R⁵ radicals; at the same time, it is optionally possible for two adjacent R⁴ substituents to form an aliphatic or aromatic or heteroaromatic ring system which may be substituted by one or more R⁵ radicals;
R⁵ is the same or different at each instance and is selected from the group consisting of H, D, F, CN, an aliphatic hydrocarbyl radical having 1 to 20 carbon atoms, and an aromatic or heteroaromatic ring system having 5 to 30 aromatic ring atoms in which one or more hydrogen atoms may be replaced by D, F, Cl, Br, I or CN and which may be substituted by one or more alkyl groups each having 1 to 4 carbon atoms; at the same time, it is possible for two or more adjacent R⁵ substituents together to form an aliphatic ring system;
excluding the following compounds:

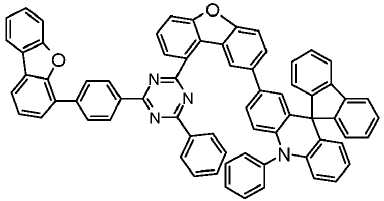

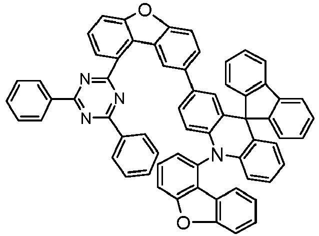

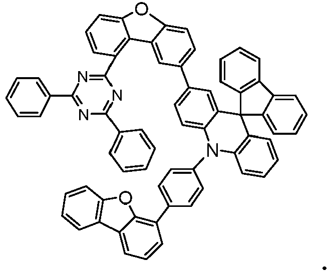

2. Compound according to Claim 1 of formula (1a) 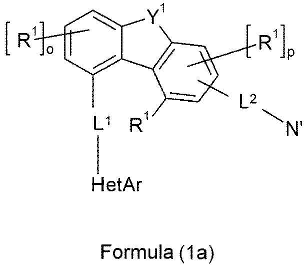 where the symbols used have the definitions given above and o is 0, 1, 2 or 3 and p is 0, 1 or 2.

3. Compound according to Claim 1 or 2 of one of the formulae (1b) to (1d) 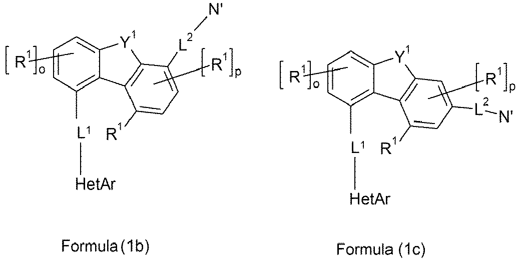 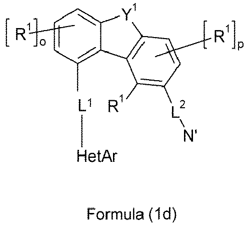 where the symbols used have the definitions given in Claims 1 and 2.

4. Compound according to one or more of Claims 1 to 3, **characterized in that** the groups of the formulae (2), (3) and (4) are selected from the groups of the formulae (2-1) to (2-10), (3-1) and (4-1) 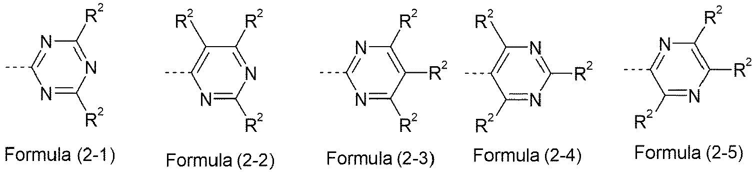 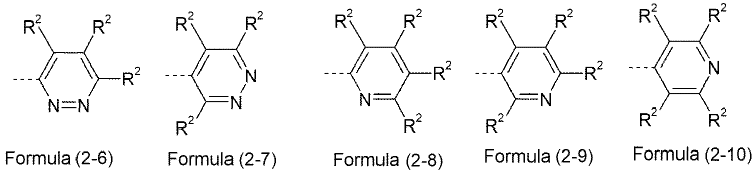 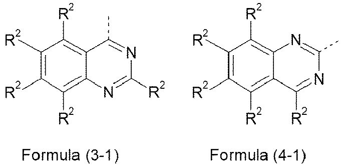 where the dotted bond represents the linkage of these groups and R² has the definitions given in Claim 1.

5. Compound according to one or more of Claims 1 to 4, **characterized in that** Ar is the same or different at each instance and is an aromatic or heteroaromatic ring system which has 6 to 18 aromatic ring atoms and may be substituted in each case by one or more R³ radicals.

6. Compound according to one or more of Claims 1 to 5, **characterized in that** the N' group is a group of the formulae (L-2) to (L-7) 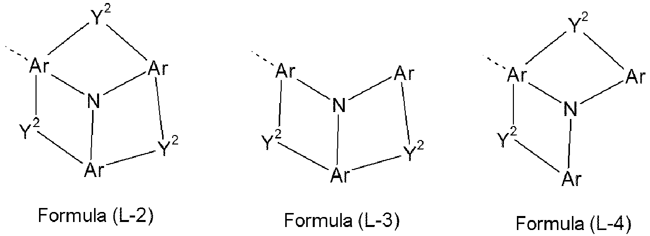 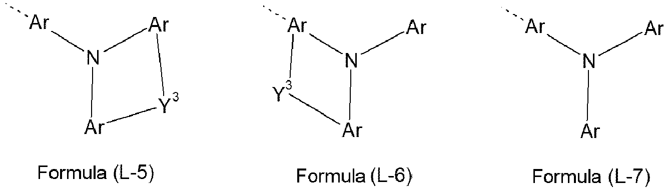 where the symbols that occur have the definitions given in Claim 1 and in addition:
Y³ is the same or different at each instance and is selected from C(R)₂, C=O,O, S, S=O, SO₂, Si(R)₂, NR, where the R radical bonded to N is not H or D.

7. Compound according to Claim 6, **characterized in that** the N' group is a group of the formula (L-7).

8. Compound according to Claim 6, **characterized in that** Y³ is the same or different at each instance and is selected from C=O,O, S, S=O, SO₂, Si(R)₂, NR, where the R radical bonded to N is not H or D.

9. Compound according to one or more of Claims 1 to 8, **characterized in that** Y¹ is O or S.

10. Process for preparing a compound according to one or more of Claims 1 to 9 proceeding from 1-halodibenzofuran or 1-halodibenzothiophene, **characterized by** the following steps:
(1) optionally converting the halogen group to a boronic acid or boronic acid derivative;
(2) introducing the HetAr or L¹-HetAr group by a coupling reaction;
(3) halogenating, especially brominating, the dibenzofuran or dibenzothiophene in the 8 position;
(4) introducing the N' group by a coupling reaction.

11. Formulation comprising at least one compound according to one or more of Claims 1 to 9 and at least one solvent.

12. Use of a compound according to one or more of Claims 1 to 9 in an electronic device.

13. Electronic device comprising at least one compound according to one or more of Claims 1 to 9.

14. Electronic device according to Claim 13 which is an organic electroluminescent device, **characterized in that** the compound according to one or more of Claims 1 to 9 is used as matrix material for fluorescent or phosphorescent emitters and/or in an electron transport layer and/or in an electron-blocking or exciton-blocking layer and/or in a hole transport layer.

## Revendications

1. Composé selon la formule (1), 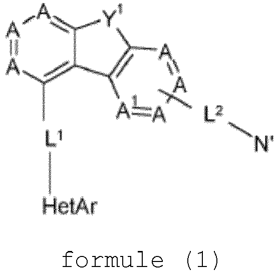 ce qui suit s'appliquant aux symboles utilisés :
A, A¹, identiques ou différents en chaque occurrence, sont CR¹ ou N, où parmi tous les A et A¹, au maximum deux groupes A et/ou A¹ par cycle représentent N et le A auquel L² est lié représente C ;
Y¹ est O, S ou NR, le radical R qui est lié à N étant différent de H ou D ;
L¹ est une simple liaison ou un système cyclique aromatique ou hétéroaromatique comportant 5 à 30 atomes de cycle aromatique, qui peut être substitué par un ou plusieurs radicaux R³ ;
L² est une simple liaison ou un système cyclique aromatique ou hétéroaromatique comportant 5 à 30 atomes de cycle aromatique, qui peut être substitué par un ou plusieurs radicaux R³ ;
N' est un groupe de formule (L-1), 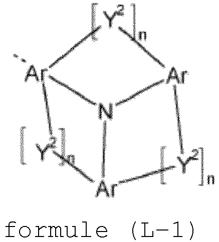
la liaison en pointillés indiquant le lien avec L2 ;
Y², identique ou différent en chaque occurrence, est choisi parmi une simple liaison, C(R)₂, C=O, O, S, S=O, SO₂, Si(R)₂, NR, le radical R qui est lié à N étant différent de H ou D ;
n, identique ou différent en chaque occurrence, est 0 ou 1, où lorsque un Y² représente une simple liaison, au moins deux n sont égaux à 1 ;
Ar, identique ou différent en chaque occurrence, est un système cyclique aromatique ou hétéroaromatique comportant 5 à 30 atomes de cycle aromatique, qui peut être substitué par un ou plusieurs radicaux R³ ;
HetAr est un groupe de formule (2), (3) ou (4), 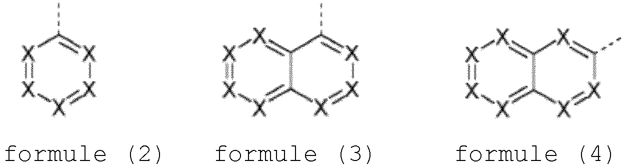 la liaison en pointillés indiquant le lien de ce groupe ;
X, identique ou différent en chaque occurrence, est CR² ou N à la condition qu'au moins un symbole X représente N ;
R, R¹, R², R³, identiques ou différents en chaque occurrence, est choisis dans le groupe constitué par H, D, F, Cl, Br, I, CN, NO₂,N(Ar¹)₂, N(R⁴)₂, C(=O)Ar¹, C(=O)R⁴, P(=O)(Ar¹)₂, P(Ar¹)₂, B(Ar¹)₂, Si(Ar¹)₃, Si(R⁴)₃, un groupe linéaire alkyle, alcoxy ou thioalkyle comportant 1 à 20 atomes de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁴, un ou plusieurs groupes CH₂ non voisins pouvant être remplacés par R⁴C=CR⁴, Si(R⁴)₂, C=O,C=NR⁴, P(=O)(R⁴), SO, SO₂, NR⁴, O, S ou CONR⁴ et un ou plusieurs atomes de H pouvant être remplacés par D, F, Cl, Br, I, CN ou NO₂,un système cyclique aromatique ou hétéroaromatique comportant 5 à 40 atomes de cycle aromatique, qui peut à chaque fois être substitué par un ou plusieurs radicaux R⁴, un groupe aryloxy ou hétéroaryloxy comportant 5 à 40 atomes de cycle aromatique, qui peut être substitué par un ou plusieurs radicaux R⁴, ou un groupe aralkyle ou hétéroaralkyle comportant 5 à 40 atomes de cycle aromatique, qui peut être substitué par un ou plusieurs radicaux R⁴ ; à cet égard éventuellement deux substituants R voisins, ou deux substituants R¹ voisins, ou deux substituants R² voisins, ou deux substituants R³ voisins, peuvent former un système cyclique aliphatique, hétéroaliphatique, aromatique ou hétéroaromatique, qui peut être substitué par un ou plusieurs radicaux R⁴ ;
Ar¹, identique ou différent en chaque occurrence, est un système cyclique aromatique ou hétéroaromatique comportant 5 à 30 atomes de cycle aromatique, qui peut être substitué par un ou plusieurs radicaux non aromatiques R⁴ ; à cet égard deux radicaux Ar¹, qui sont liés au même atome de N, atome de P ou atome de B, peuvent être pontés l'un avec l'autre, également par simple liaison ou un pont choisi parmi N(R⁴), C(R⁴)₂, O ou S ;
R⁴, identique ou différent en chaque occurrence, est choisi dans le groupe constitué par H, D, F, Cl, Br, I, CN, NO₂,N(R⁵)₂, C(=O)R⁵, Si(R⁵)₃, un groupe linéaire alkyle, alcoxy ou thioalkyle comportant 1 à 20 atomes de C ou un groupe ramifié ou cyclique alkyle, alcoxy ou thioalkyle comportant 3 à 20 atomes de C ou un groupe alcényle comportant 2 à 20 atomes de C, qui peut à chaque fois être substitué par un ou plusieurs radicaux R⁵, un ou plusieurs groupes CH₂ non voisins pouvant être remplacés par R⁵C=CR⁵, Si(R⁵)₂, C=O,C=NR⁵, P(=O)(R⁵), SO, SO₂, NR⁵, O, S ou CONR⁵ et un ou plusieurs atomes de H pouvant être remplacés par D, F, Cl, Br, I, CN ou NO₂,un système cyclique aromatique ou hétéroaromatique comportant 5 à 40 atomes de cycle aromatique, qui peut à chaque fois être substitué par un ou plusieurs radicaux R⁵, un groupe aryloxy ou hétéroaryloxy comportant 5 à 40 atomes de cycle aromatique, qui peut être substitué par un ou plusieurs radicaux R⁵, ou un groupe aralkyle ou hétéroaralkyle comportant 5 à 40 atomes de cycle aromatique qui peut être substitué par un ou plusieurs radicaux R⁵ ; à cet égard deux substituants voisins R⁴ peuvent éventuellement former un système cyclique aliphatique ou aromatique ou hétéroaromatique, qui peut être substitué par un ou plusieurs radicaux R⁵ ;
R⁵, identique ou différent en chaque occurrence, est choisi dans le groupe constitué par H, D, F, CN, un radical hydrocarboné aliphatique comportant 1 à 20 atomes de C ou un système cyclique aromatique ou hétéroaromatique comportant 5 à 30 atomes de cycle aromatique, dans lequel un ou plusieurs atomes de H peuvent être remplacés par D, F, Cl, Br, I ou CN et qui peut être substitué par un ou plusieurs groupes alkyle comportant à chaque fois 1 à 4 atomes de carbone ; à cet égard deux substituants R⁵ voisins ou plus peuvent former ensemble un système cyclique aliphatique ,
les composés suivants étant exclus :

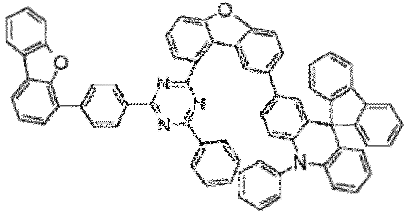

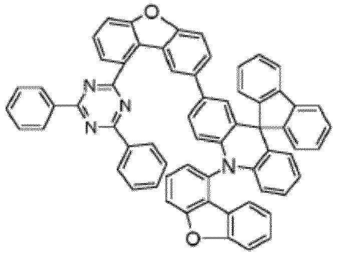

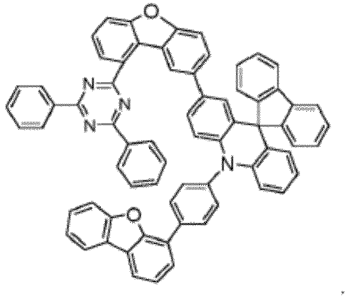

2. Composé selon la revendication 1 selon la formule (1a), 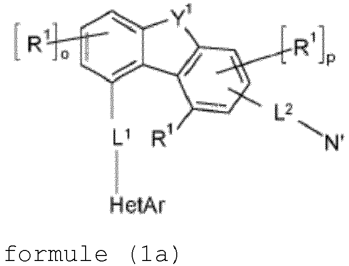 les symboles utilisés présentant les significations mentionnées ci-dessus et o représentant 0, 1, 2 ou 3 et p représentant 0, 1 ou 2.

3. Composé selon la revendication 1 ou 2 selon l'une des formules (1b) à (1d), 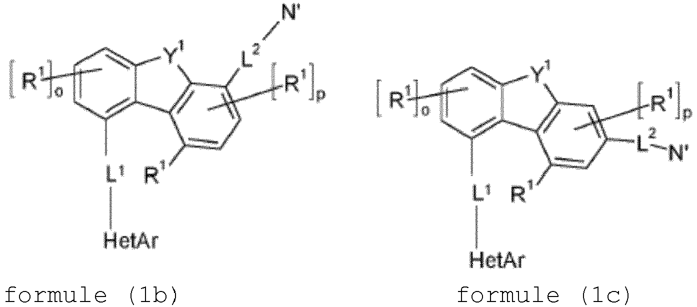 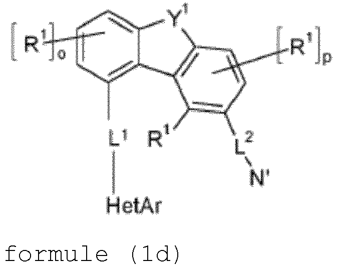 les symboles utilisés présentant les significations mentionnées dans les revendications 1 et 2.

4. Composé selon l'une ou plusieurs des revendications 1 à 3, **caractérisé en ce que** les groupes des formules (2), (3) et (4) sont choisis parmi les groupes des formules (2-1) à (2-10), (3-1) et (4-1), 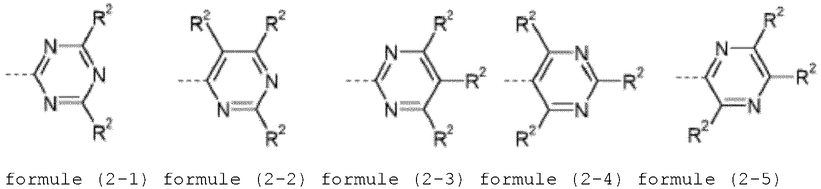 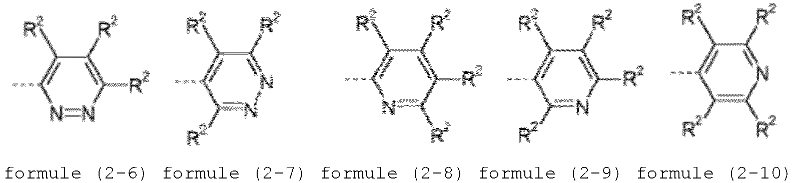 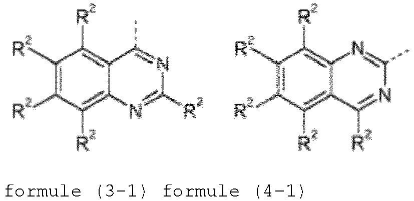 la liaison en pointillés indiquant le lien de ces groupes et R² présentant les significations mentionnées dans la revendication 1.

5. Composé selon l'une ou plusieurs des revendications 1 à 4, **caractérisé en ce que** Ar, identique ou différent en chaque occurrence, représente un système cyclique aromatique ou hétéroaromatique comportant 6 à 18 atomes de cycle aromatique, qui peut à chaque fois être substitué par un ou plusieurs radicaux R³.

6. Composé selon l'une ou plusieurs des revendications 1 à 5, **caractérisé en ce que** le groupe N' est un groupe parmi les formules (L-2) à (L-7), 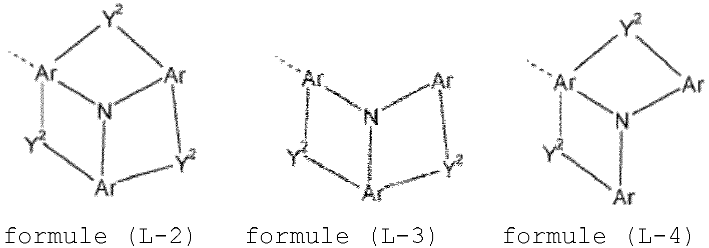 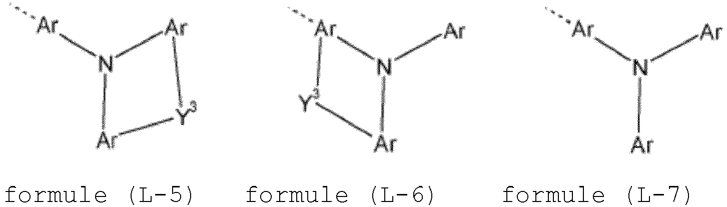 les symboles apparaissant présentant les significations mentionnées dans la revendication 1 et de plus ce qui suit s'appliquant :
Y³, identique ou différent en chaque occurrence, est choisi parmi C(R)₂, C=O, O, S, S=O, SO₂, Si(R)₂, NR,
le radical R qui est lié à N étant différent de H ou D.

7. Composé selon la revendication 6, **caractérisé en ce que** le groupe N' est un groupe de formule (L-7).

8. Composé selon la revendication 6, **caractérisé en ce que** Y³, identique ou différent en chaque occurrence, est choisi parmi C=O, O, S, S=O, SO₂, Si(R)₂, NR, le radical R qui est lié à N étant différent de H ou D.

9. Composé selon l'une ou plusieurs des revendications 1 à 8, **caractérisé en ce que** Y¹ représente O ou S.

10. Procédé pour la préparation d'un composé selon l'une ou plusieurs des revendications 1 à 9 à partir d'un 1-halogéno-dibenzofuranne ou d'un 1-halogéno-dibenzothiophène, **caractérisé par** les étapes suivantes :
(1) transformation éventuelle du groupe halogéno en un acide boronique ou un dérivé d'acide boronique ;
(2) introduction du groupe HetAr ou L¹-HetAr par une réaction de couplage ;
(3) halogénation, en particulier bromation, du dibenzofuranne ou du dibenzothiophène en position 8 ;
(4) introduction du groupe N' par une réaction de couplage.

11. Formulation, contenant au moins un composé selon l'une ou plusieurs des revendications 1 à 9 et au moins un solvant.

12. Utilisation d'un composé selon l'une ou plusieurs des revendications 1 à 9 dans un dispositif électronique.

13. Dispositif électronique contenant au moins un composé selon l'une ou plusieurs des revendications 1 à 9.

14. Dispositif électronique selon la revendication 13, qui est un dispositif d'électroluminescence organique, **caractérisé en ce que** le composé selon l'une ou plusieurs des revendications 1 à 9 est utilisé en tant que matériau de matrice pour des émetteurs fluorescents ou phosphorescents et/ou dans une couche de transport d'électrons et/ou dans une couche de blocage électrons ou de blocage d'excitons et/ou dans une couche de transport de trous.
